# EUROPEAN PATENT SPECIFICATION

(11) **EP 2 714 070 B1**
(45) Date of publication and mention of the grant of the patent: **20.03.2024**
(21) Application number: 12793107.9
(22) Date of filing: 04.06.2012
(51) Int. Cl.: A61K 38/26, C07K 14/605, A61P 3/10, A61P 7/12, A61K 47/60, A61P 3/04, A61P 3/08, A61P 43/00

(54) **LONG-ACTING GLP-1/GLUCAGON RECEPTOR AGONISTS**
LANGWIRKENDE GLP-1/GLUCAGONREZEPTORAGONISTEN
AGONISTES DE RÉCEPTEUR DU GLP-1/GLUCAGON À ACTION LONGUE

(30) Priority: 02.06.2011 US 201161492448 P; 16.04.2012 US 201261624589 P
(43) Date of publication of application: 09.04.2014
(60) Divisional application: 21204106.5
(73) Proprietor: OPKO Biologics Ltd., Kiryat Gat 8211804 (IL)
(72) Inventor: FIMA, Udi, Eyal, 84833 Beer-Sheva (IL); HERSHKOVITZ, Oren, 75256 Rishon Lezion (IL)
(74) Representative: Pearl Cohen Zedek Latzer Baratz UK LLP
(86) International application number: PCT/US2012/040744
(87) International publication number: WO 2012/167251

(56) References cited:
- WO-A1-2011/087671
- WO-A2-2010/107256
- US-A1- 2006 171 920
- US-A1- 2010 144 617
- Oren Hershkovitz et al.: "MOD-6030, a long-acting dual GLP-1/Glucagon agonist improves glycemic control and induces a prolonged anti-obesity effect in Diet Induced Obesity mice, with a potential once weekly injection in humans", , 19 April 2012 (2012-04-19), XP002734249, Retrieved from the Internet: URL:https://www.gtcbio.com/preview/diabete s2012/abstracts.htm [retrieved on 2015-01-09]
- Oren Hershkovitz et al.: "MOD-6030, A Long-Acting Dual GLP-1/Glucagon Agonist Improves Glycemic Control andInduces a Prolonged Anti-Obesity Effect in DietInduced Obesity Mice, with a Potential Once WeeklyInjection in Humans", , 19 April 2012 (2012-04-19), page 17PP, XP002734250, Retrieved from the Internet: URL:https://www.gtcbio.com/preview/diabete s_summit.pdf [retrieved on 2015-01-09]
- Anonymous: "Long Acting Reversible PEGylated Oxyntomodulin - MOD-6030", , 19 April 2012 (2012-04-19), XP002734251, Retrieved from the Internet: URL:http://www.sec.gov/Archives/edgar/data /1268659/000114420412022748/v309878_ex99-1 .htm [retrieved on 2015-01-09]
- DRUCE MARALYN R ET AL: "Investigation of Structure-Activity Relationships of Oxyntomodulin (Oxm) Using Oxm Analogs", ENDOCRINOLOGY, vol. 150, no. 4, 1 April 2009 (2009-04-01) , pages 1712-1721, XP009116621, THE ENDOCRINE SOCIETY, US ISSN: 0013-7227, DOI: 10.1210/EN.2008-0828 [retrieved on 2008-12-12]
- LAURIE L. BAGGIO ET AL: "Oxyntomodulin and glucagon-like peptide-1 differentially regulate murine food intake and energy expenditure", GASTROENTEROLOGY, vol. 127, no. 2, 1 August 2004 (2004-08-01), pages 546-558, XP055161066, ISSN: 0016-5085, DOI: 10.1053/j.gastro.2004.04.063
- Parlevliet, Edwin T, et al.: "Oxyntomodulin increases insulin secretion but does not affect insulin sensitivity in high-fat-fed C57Bl/6 mice", EBSCO Host Connection , June 2007 (2007-06), XP002734275, Retrieved from the Internet: URL:http://connection.ebscohost.com/c/arti cles/25821756/oxyntomodulin-increases-insu lin-secretion-but-does-not-affect-insulin- sensitivity-high-fat-fed-c57b1-6-mice [retrieved on 2015-01-08]
- BIANCHI ELISABETTA ET AL: "A PEGylated analog of the gut hormone oxyntomodulin with long-lasting antihyperglycemic, insulinotropic and anorexigenic activity", BIOORGANIC & MEDICINAL CHEMISTRY, vol. 21, no. 22, 19 September 2013 (2013-09-19), pages 7064-7073, XP028754791, ISSN: 0968-0896, DOI: 10.1016/J.BMC.2013.09.016

## Description

### FIELD OF INVENTION

Pegylated and reverse pegylated oxyntomodulin including pharmaceutical compositions comprising the same and medical uses thereof are disclosed.

### BACKGROUND OF THE INVENTION

Proteins and especially short peptides are susceptible to denaturation or enzymatic degradation in the blood, liver or kidney. Accordingly, proteins typically have short circulatory half-lives of several hours. Because of their low stability, peptide drugs are usually delivered in a sustained frequency so as to maintain an effective plasma concentration of the active peptide. Moreover, since peptide drugs are usually administered by infusion, frequent injection of peptide drugs cause considerable discomfort to a subject. Thus, there is a need for technologies that will prolong the half-lives of therapeutic proteins and peptides while maintaining a high pharmacological efficacy thereof. Such desired peptide drugs should also meet the requirements of enhanced serum stability, high activity and a low probability of inducing an undesired immune response when injected into a subject.

Unfavorable pharmacokinetics, such as a short serum half-life, can prevent the pharmaceutical development of many otherwise promising drug candidates. Serum half-life is an empirical characteristic of a molecule, and must be determined experimentally for each new potential drug. For example, with lower molecular weight protein drugs, physiological clearance mechanisms such as renal filtration can make the maintenance of therapeutic levels of a drug unfeasible because of cost or frequency of the required dosing regimen.

The gastrointestinal tract is responsible on synthesize and releasing of many peptide hormones that regulate eating behavior including pancreatic protein (PP), glucagon-like peptide 1 (GLP-1), peptide YY (PYY) and Oxyntomodulin (OXM). OXM arises from a tissue-specific post-transitional processing of proglucagon in the intestine and the CNS. It contains 37 amino acids, including the complete glucagon sequence with a C-terminal basic octapeptide extension that was shown to contribute to the properties of OXM both in-vitro and in-vivo but was not alone sufficient for the effects of the peptide. In response to food ingestion, OXM is secreted by intestinal L cells into the bloodstream proportionally to the meal caloric content.

OXM enhances glucose clearance via stimulation of insulin secretion after both oral and intraperitoneal administration. It also regulates the control of food intake. Intracerebroventricular (ICV) and intranuclear injection of OXM into the paraventricular and arcuate nuclei (ARC) of the hypothalamus inhibits re-feeding in fasting rats (Dakin et al. 2001; Dakin et al. 2004). This inhibition has also been demonstrated in freely fed rats at the start of the dark phase. Moreover, peripheral administration of OXM dose-dependently inhibited both fast-induced and dark-phase food intake (Dakin et al. 2004).

US 2010/0144617 describes derivatives of oxyntomodulin that have been mutated to provide a stabilized oxyntomodulin that does not exhibit glucagon receptor agonist activity.

A new conceptual approach termed reversible pegylation was previously described (PCT Publication No. WO 98/05361; Gershonov et al., 2000), for prolonging the half-life of proteins and peptides. According to this technology, prodrugs are prepared by derivatizing the drug with functional groups that are sensitive to bases and removable under mild basic conditions such as physiological conditions. The derivatization includes a substitution of at least one amino, hydroxyl mercapto and/or carboxyl groups of the drug molecule with a linker such as 9-fluorenylmethoxycarbonyl (Fmoc) and 2-sulfo-9-fluorenylmethoxycarbonyl (FMS), to which a group of Polyethylene glycol (PEG) moiety is attached. The link between the PEG moiety and the drug is not direct but rather both residues are linked to different positions of the scaffold FMS or Fmoc structures that are highly sensitive to basic conditions. US 2006/0171920 describes methods for the reversible pegylation of insulin, an interferon, a PYY agonist, an exendin, atrial natriuretic peptide, human growth hormone, erythropoietin, TNF-α, calcitonin, gonadotropin releasing hormone, hirudin, glucagon, and a monoclonal antibody fragment.

Hershkovitz *et al.* disclose MOD-6030, a long acting dual GLP-1/Glucagon agonist improving glycemic control and inducing a prolonged anti-obesity effect in diet induced obesity mice with a potential once weekly injection in humans (19 April 2012, XP002734249, https://www.gtcbio.com/preview/diabetes2012/abstracts.htm). Prolor Biotech discloses a long acting reversible PEGylated Oxyntomodulin - MOD-6030 (XP002734251, http://www.sec.gov/Archives/edgar/data/1268659/000114420412022748/v309878_ex99-1.htm).

The present invention relates to OXM derivative in which the half-life of the peptide is prolonged utilizing the reversible pegylation technology.

### SUMMARY OF THE INVENTION

The present invention provides a compound of the formula (X)ₙ-Y, wherein Y is dual GLP-1/Glucagon receptor agonist consisting of an oxyntomodulin (OXM) peptide having the amino acid sequence of SEQ ID NO: 1 and bearing a free amino or hydroxyl group, and wherein X is a radical of formula (i) 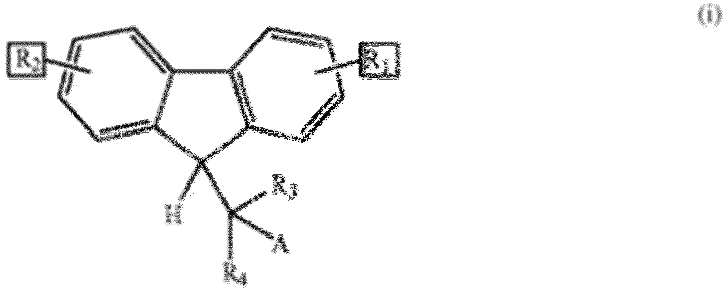 wher ein
R₁ is a polyethylene glycol (PEG) moiety;
R₂ is either hydrogen or is -SO₃H at position 2 of the fluorene ring;
R₃ and R₄ are each hydrogen;
A is -OCO-, wherein the radical of formula (i) is linked to an amino or hydroxyl group of the drug Y; and
n is an integer of at least one.

In an embodiment, the PEG moiety is a linear PEG.

In an embodiment, the PEG moiety is a branched PEG.

In an embodiment, the PEG moiety is a 30 kDa, 40 kDa or 60 kDa PEG.

In an embodiment, the compound is prepared from a MAL-Fmoc-NHS having the formula: 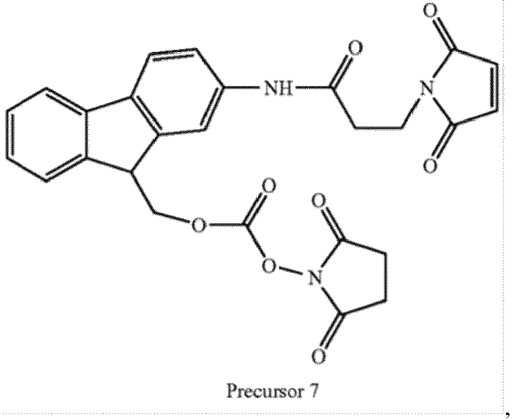 wherein the compound is designated as (PEG-Fmoc-)n-OXM.

In an embodiment, Y is linked to the Fmoc radical through an amino group, which in an embodiment is the amino terminus of the oxyntomodulin.

In an embodiment, the compound is prepared from a MAL-FMS-NHS having the formula: 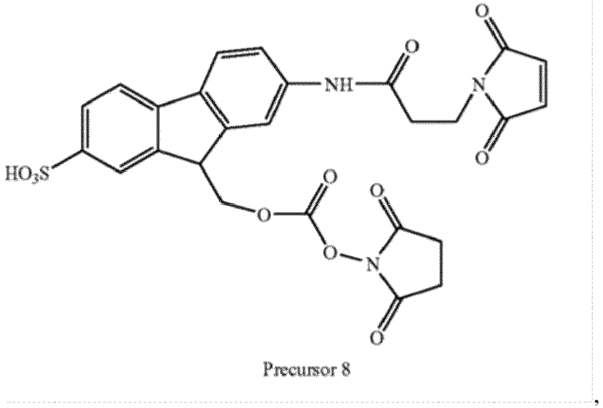 wherein said compound is designated as (PEG-FMS-)n-OXM.

In an embodiment, Y is linked to the FMS radical through an amino group, which in an embodiment is the amino terminus of the oxyntomodulin.

In an embodiment, the PEG is linked to the FMS or Fmoc radical through a sulfhydryl group.

The present invention also provides a composition comprising a compound of the present invention, and a pharmaceutical acceptable carrier.

The present invention further provides a method for the preparation of oxyntomodulin having an extended biological half-life, consisting of the step of conjugating oxyntomodulin, a polyethylene glycol polymer (PEG polymer) and 9-fluorenylmethoxycarbonyl (Fmoc) or 2-sulfo-9-fluorenylmethoxycarbonyl (FMS) in a molar ratio of 1:1:0.5 to 1:1:3.5, to form a compound of the invention.

The present invention provides a compound or a composition of the invention for use in inducing glucose tolerance and/or improving glycemic control, or for use in reducing food intake and/or reducing body weight, in a subject in need thereof.

The present invention also provides a compound of the invention for use in treating diabetes mellitus, diabetes mellitus associated with obesity, obesity, an eating disorder, or a metabolic disorder, in a subject.

The present invention also provides a compound or a composition of the invention for use as a medicament.

In an embodiment, R₂ is -SO₃H at position 2 of the fluorene ring, and the PEG moiety is a 40 kDa PEG.

Other features and advantages of the present invention will become apparent from the following detailed description, examples and figures. It should be understood, however, that the detailed description and the specific examples while indicating preferred embodiments of the invention are given by way of illustration only, since various changes and modifications within the scope of the claims will become apparent to those skilled in the art from this detailed description.

### BRIEF DESCRIPTION OF THE DRAWINGS

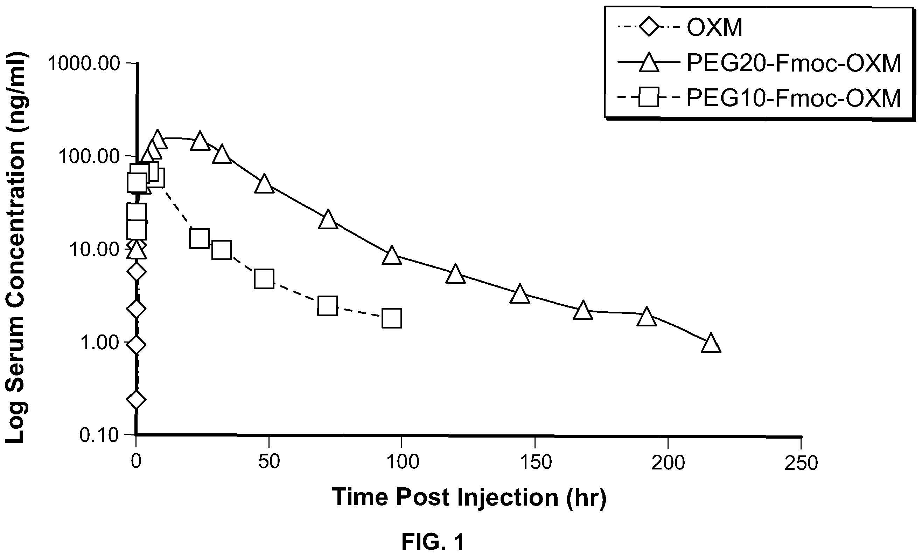

**Figure 1** is a graph showing the pharmacokinetic profile of OXM, PEG₁₀-Fmoc-OXM and PEG₂₀-Fmoc-OXM in male rats. Rats received a single SC bolus injection of native OXM (62nmol/kg), PEG₁₀-Fmoc-OXM (containing 278µg/kg OXM peptide) or PEG₂₀-Fmoc-OXM (containing 62nmol/kg OXM peptide) in 0.5 ml PBS buffer. Serum samples were collected from the jugular vein at specified time points and OXM concentration was analyzed using OXM Elisa kit (Bachem, Switzerland).

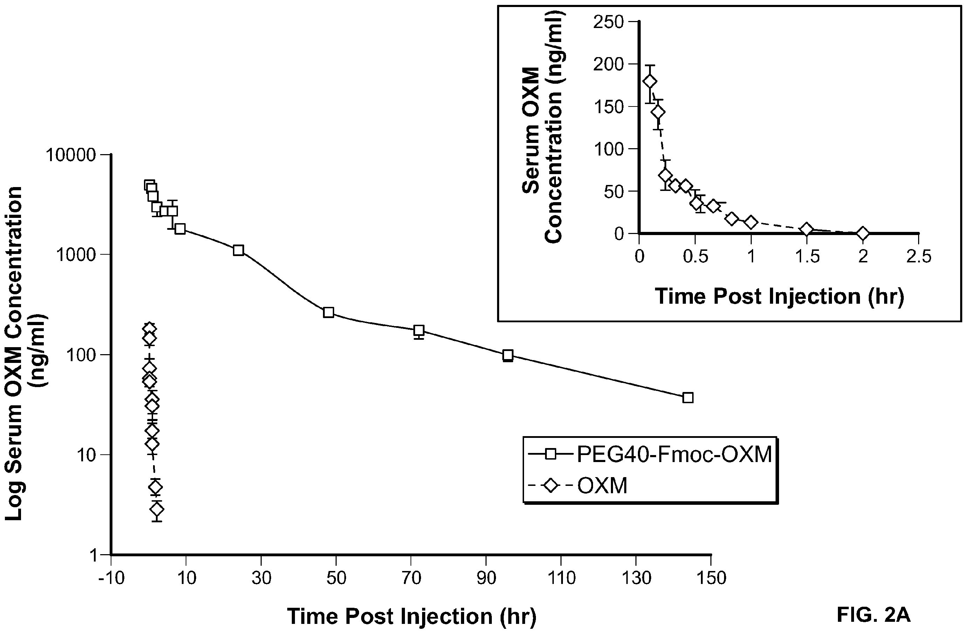

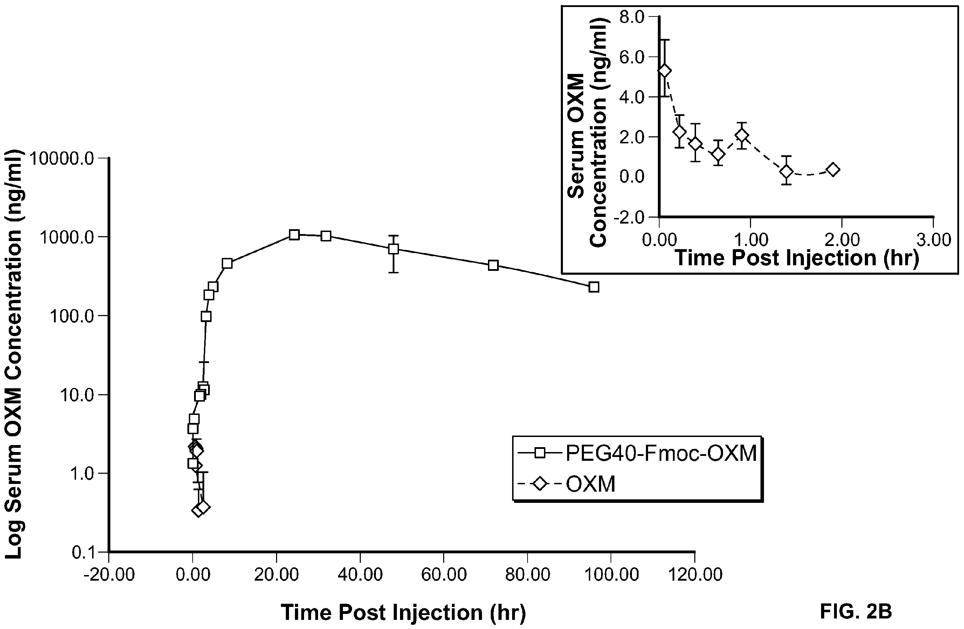

**Figure 2** are graphs showing the pharmacokinetic profile of OXM and PEG₄₀-Fmoc-OXM in male rats. Rats received a single IV bolus (A) or SC (B) injection of native OXM (62nmol/kg) or PEG₄₀-Fmoc-OXM (containing 62nmol/kg body weight OXM peptide) in 0.5 ml PBS buffer. Serum samples were collected from the jugular vein at specified time points and OXM concentration was analyzed using OXM Elisa kit (Bachem, Switzerland). The overlay insert highlight the OXM profile which is apparent in the first two hours after administration.

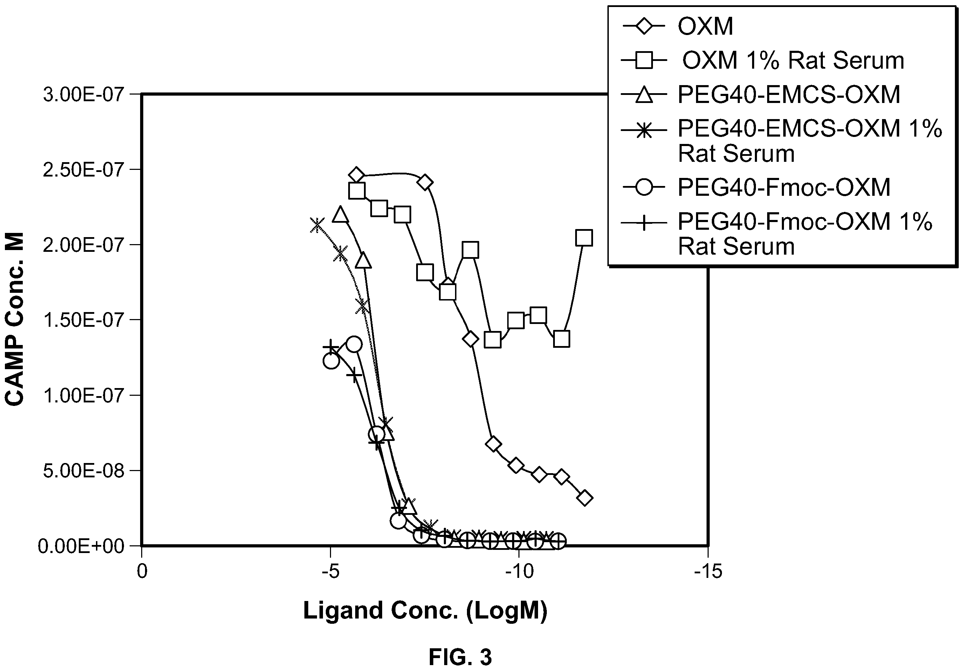

**Figure 3** is a graph showing the in-vitro activity of native OXM, PEG₄₀-Fmoc-OXM and PEG₄₀-EMCS-OXM. CHO-K1 cells over-expressing GLP-1 receptor (Millipore HTS163C2) were seeded in 96 wells half-area white at a density of 200,000 cells/ml and incubated for 24 hours at 37°C. The cells were incubated with escalating concentrations of OXM (ALMAC), PEG40-EMCS-OXM and PEG40-Fmoc-OXM with or without Rat serum 1% (Bio reclamation). Cells cAMP concentrations were quantified by HTRF assay (Cisbio 62AM4PEB) and EC50 parameter was analyzed by PRISM software.

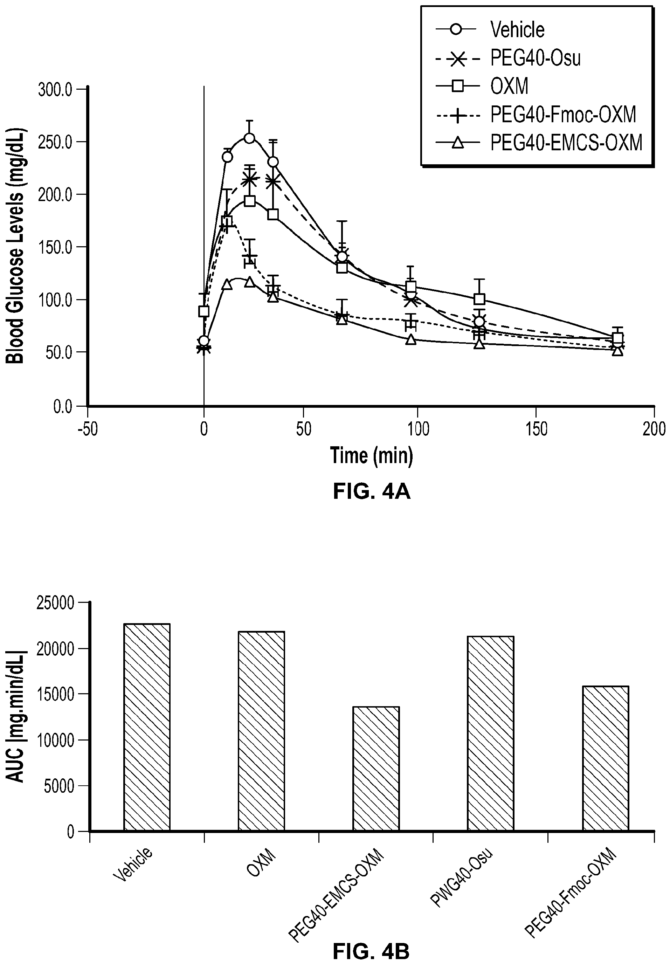

**Figure 4** are graphs showing the induction of glucose tolerance in mice with native OXM and PEG₄₀-Fmoc-OXM and PEG₄₀-EMCS-OXM as measured by IP glucose tolerance test (IPGTT). C57BL/6 Mice were fasted overnight and then injected IP with PBS (vehicle), PEG₄₀-Osu as control (546nmol/kg), native OXM (333nmol/kg), PEG₄₀-Fmoc-OXM (202nmol/kg peptide content) and PEG₄₀-EMCS-OXM (333nmol/kg). Glucose (1.5gr/kg) was administrated IP either 15min after test article administration (vehicle, OXM and PEG₄₀-Osu) or 120 min after PEG₄₀-Fmoc-OXM administration. Blood glucose levels were measured by tail vein sampling prior to glucose administration and 10, 20, 30, 60 and 120 min after glucose administration using a handheld glucometer. Graph (A) provides the blood glucose profile and graph (B) shows the glucose AUC.

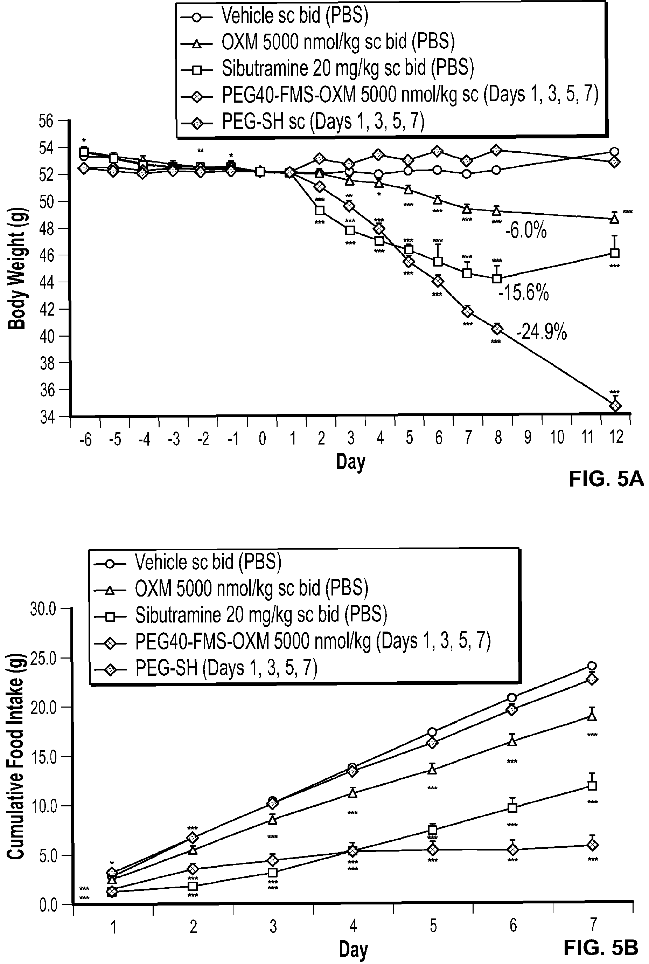

**Figure 5** are graphs showing the effect of SC administration OXM (b.i.d) and PEG40-FMS-OXM (days 1, 3, 5, 7) on body weight (A) and cumulative food intake (B) in male C57BL/6J mice exhibiting diet induced obesity. Data are adjusted means (n = 10). SEMs are calculated from the residuals of the statistical model. Mice were dosed for 7 days starting on Day 1. Data analyzed by ANCOVA with body weight on Day 1 as covariate followed by Williams' test (OXM in PBS) or multiple t test (sibutramine and PEG40-FMS-OXM) vs appropriate vehicle. Significant differences vs. appropriate vehicle: *p<0.05, **p<0.01, ***p<0.001. Percentage values indicate difference from appropriate vehicle group on Day 8 (i.e. after 7 days dosing).

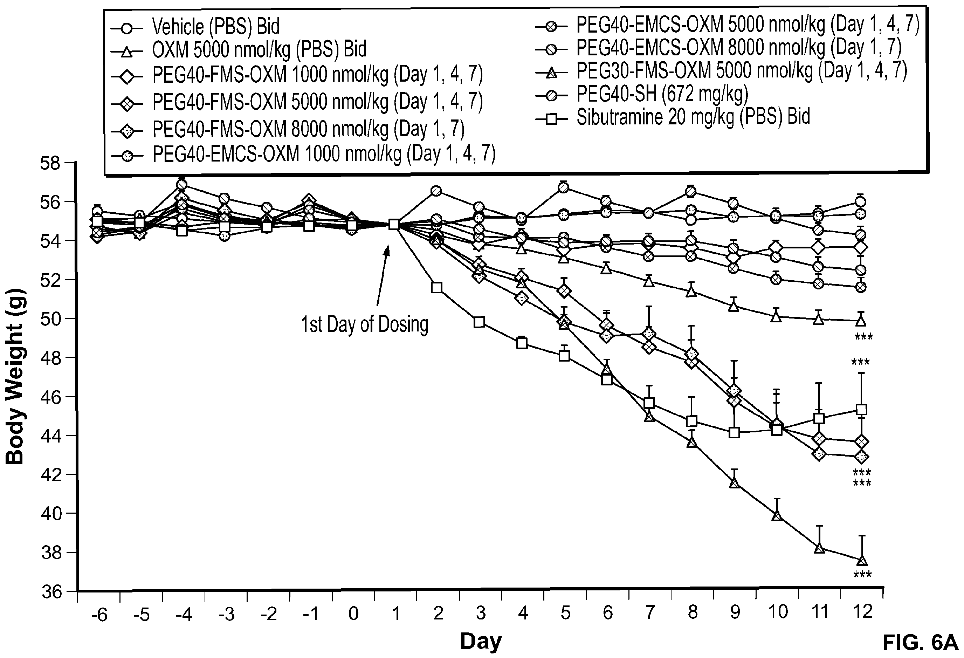

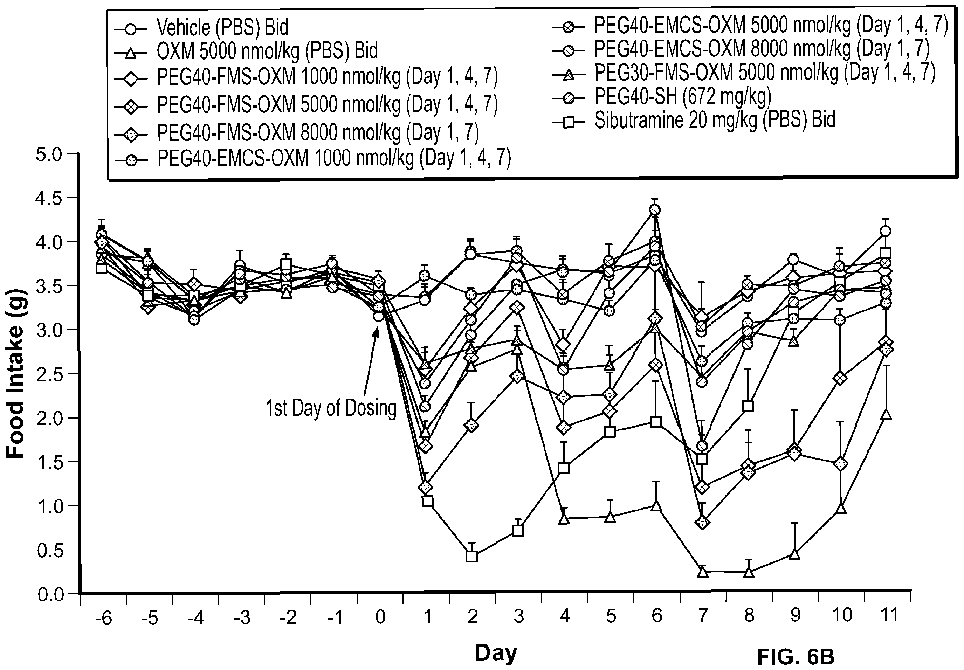

**Figure 6** is a graph showing effect of SC administration OXM (b.i.d) and covalently bound pegylated OXM PEG40-EMCS-OXM (1000nmol/kg and 5000nmol/kg on Days 1, 4 and 7 or 8000nmol/kg on Days 1 and 7), PEG40-FMS-OXM (1000nmol/kg and 5000nmol/kg on Days 1, 4 and 7 or 8000nmol/kg on Days 1 and 7) and PEG30-FMS-OXM (5000nmol/kg on Days 1, 4 and 7 ) on body weight (A) and food intake (B) in male C57BL/6J mice exhibiting diet induced obesity. Data are adjusted means (n = 10). SEMs are calculated from the residuals of the statistical model. Mice were dosed for 7 days starting on Day 1.

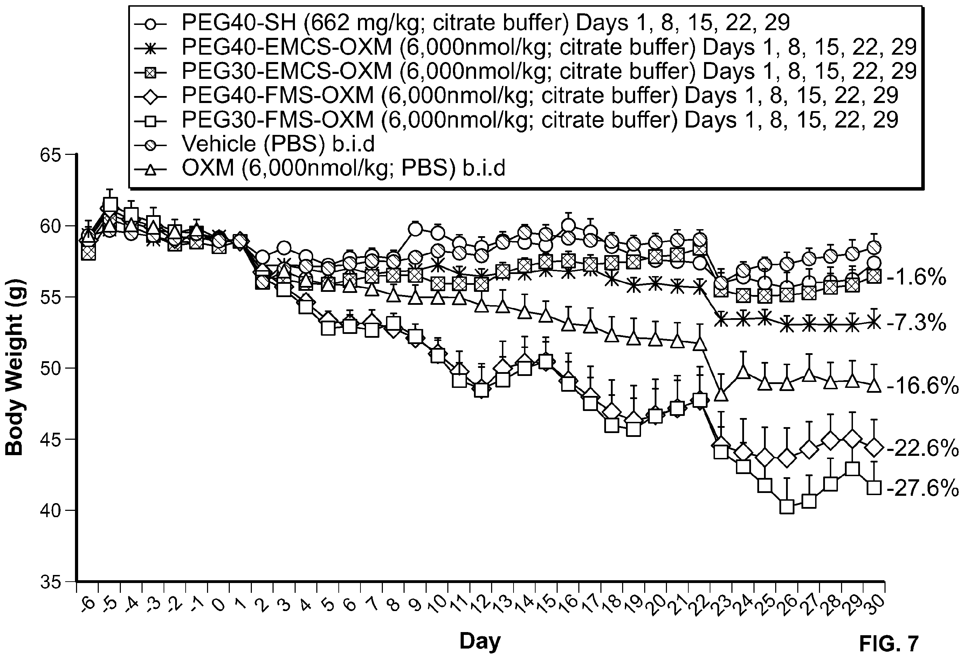

**Figure 7** shows the effects of reversible PEGylated OXM Administration on body weight in Diet Induced Obesity (DIO) Mice. During the first week of single housing (handling period), animals began a once-daily handling protocol and during the second week (baseline period), they were dosed with the appropriate vehicle b.i.d. or once a week as they were dosed during the treatment period) by a subcutaneous route. 7 groups (n=8) of DIO mice were dosed for 29 days as follows: A. PEG40-SH (662 mg/kg), B. PEG40-EMCS-OXM (6,000nmol/kg), C. PEG30-EMCS-OXM (6,000nmol/kg), D. PEG40-FMS-OXM (6,000nmol/kg), E. PEG30-FMS-OXM (6,000nmol/kg), F. Vehicle (PBS), and G. OXM (6,000nmol/kg; PBS). During the baseline and the treatment period food intake, water intake and body weight were recorded daily. Weekly administration of PEG40-FMS-OXM or PEG30-FMS-OXM significantly reduced body weight in Diet Induced Obesity (DIO) mice.

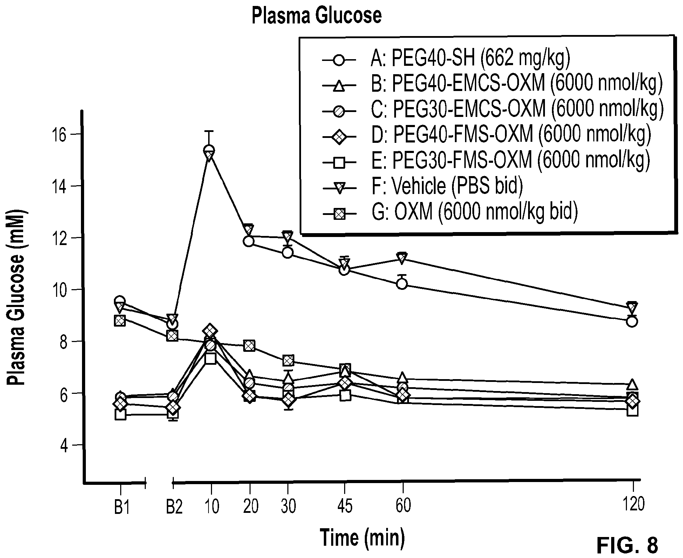

**Figure 8** shows the acute effects of reversible PEGylated OXM administration on glucose tolerance in Diet Induced Obesity (DIO) Mice. On day 1 after the start of drug or vehicle administration, all the mice were overnight fasted. On day 2, the mice underwent an oral glucose tolerance test (OGTT). Each animal were dosed with vehicle or test compound and 60 minutes later were dosed with D-glucose (2 g/kg po). Baseline blood samples were taken immediately prior to dosing with vehicle or test compound (B1) and immediately before the glucose load (B2). Further blood samples were taken 10, 20, 30, 45, 60 and 120 minutes post glucose administration. All blood samples (approximately 20µl) were taken from the tail vein. Plasma samples were prepared and assayed for glucose (n = 2) and insulin (n = 1) using the Thermoelectron Infinity glucose reagent (TR15421) and Alpco mouse ultrasensitive insulin ELISA (80-INSMSU-E10), respectively.

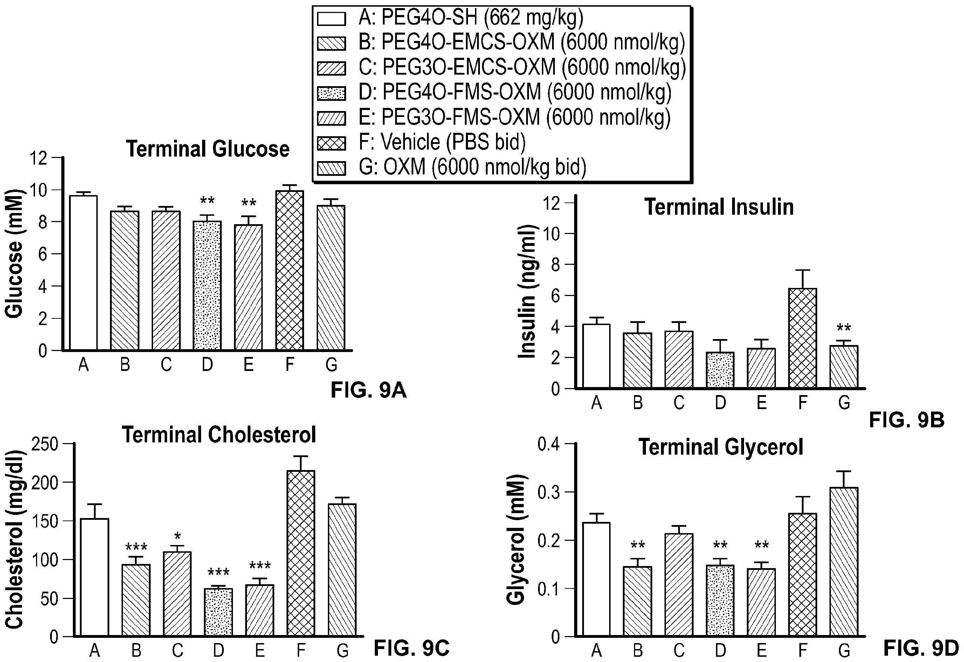

**Figure 9** shows the effects of reversible PEGylated OXM administration on terminal glucose, glycerol, cholesterol and insulin in Diet Induced Obesity (DIO) Mice. Terminal plasma samples were collected (24 hours after the final dose of test or control compound on Day 29) by cardiac puncture and assayed for insulin, glucose and cholesterol using the mouse ultrasensitive insulin ELISA (80-INSMSU-E10), Thermoelectron Infinity glucose reagent (TR15421) and Thermoelectron Infinity cholesterol reagent (TR13421).

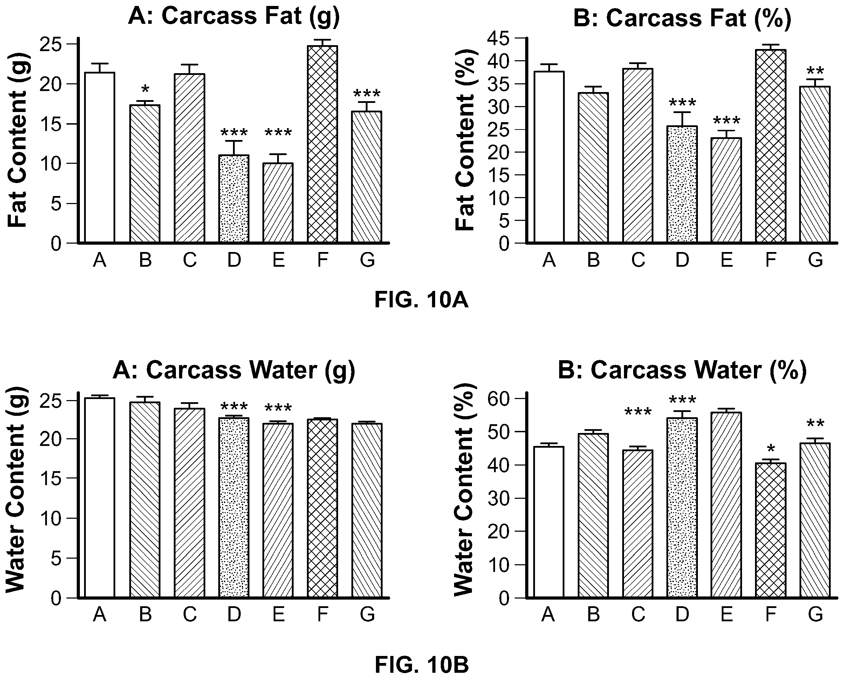

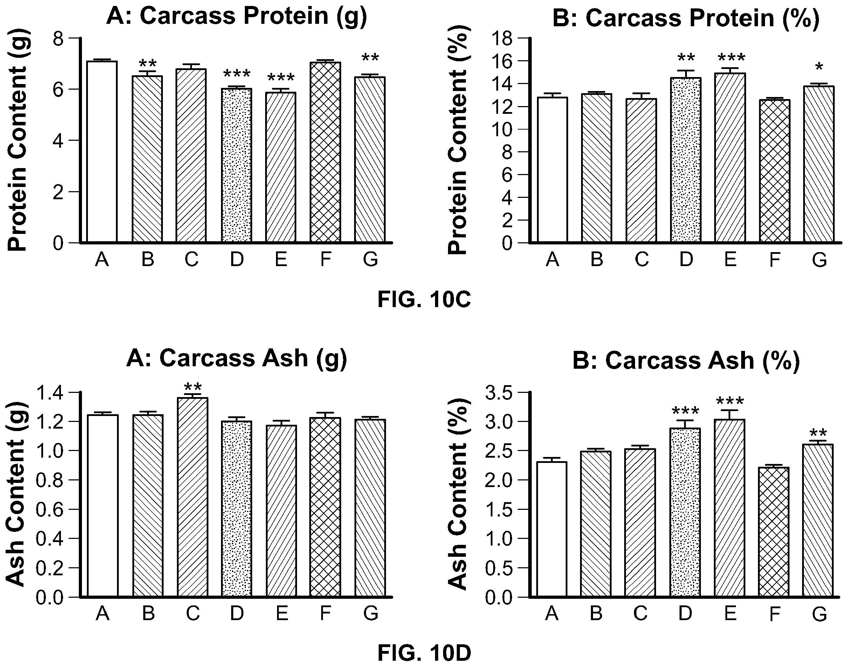

**Figure 10** shows the effects of reversible PEGylated OXM administration on terminal body composition analysis of fat, water, protein and ash (bone) in Diet Induced Obesity (DIO) Mice. Body fat (A), water (B), protein (C), and ash levels (D) of DIO mouse carcasses were determined using standard chemical analysis techniques. The treatment groups were as follows: A. PEG40-SH (662 mg/kg), B. PEG40-EMCS-OXM (6,000nmol/kg), C. PEG30-EMCS-OXM (6,000nmol/kg), D. PEG40-FMS-OXM (6,000nmol/kg), E. PEG30-FMS-OXM (6,000nmol/kg), F. Vehicle (PBS), and G. OXM (6,000nmol/kg; PBS).

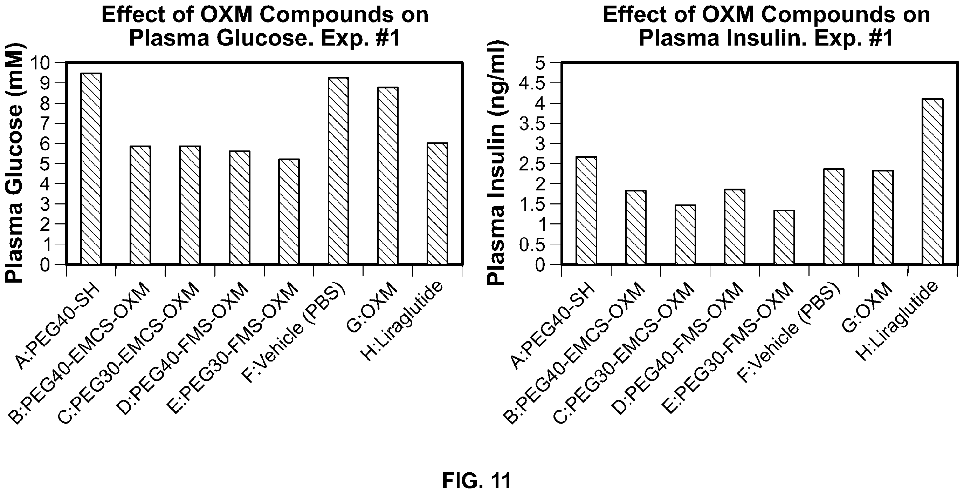

**Figure 11** shows that administration of PEG-OXM variants PEG40-EMCS-OXM, PEG30-EMCS-OXM, PEG40-FMS-OXM, PEG30-FMS-OXM produced marked and significant reductions in fasting glucose and fasting plasma insulin when compared to controls.

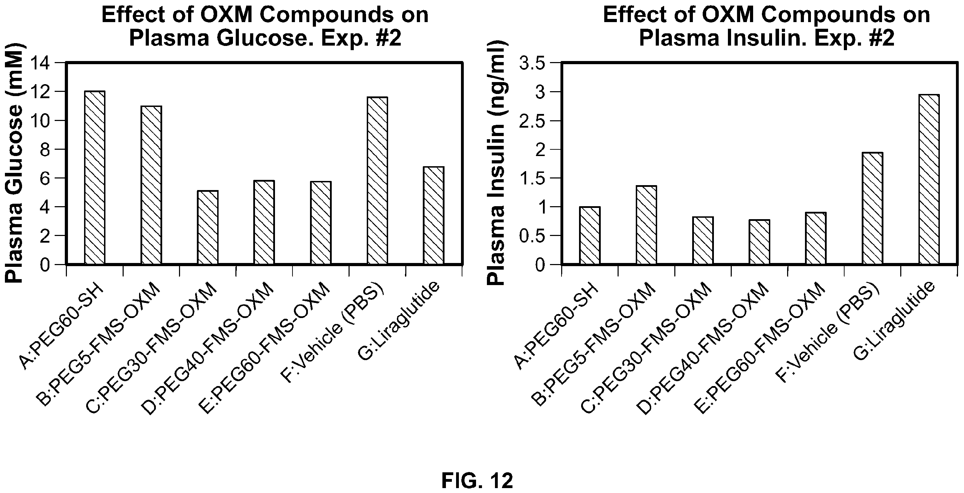

**Figure 12** shows that administration of PEG-OXM variants PEG30-FMS-OXM, PEG40-FMS-OXM and PEG60-FMS-OXM produced marked and significant reductions in fasting glucose and fasting plasma insulin when compared to controls.

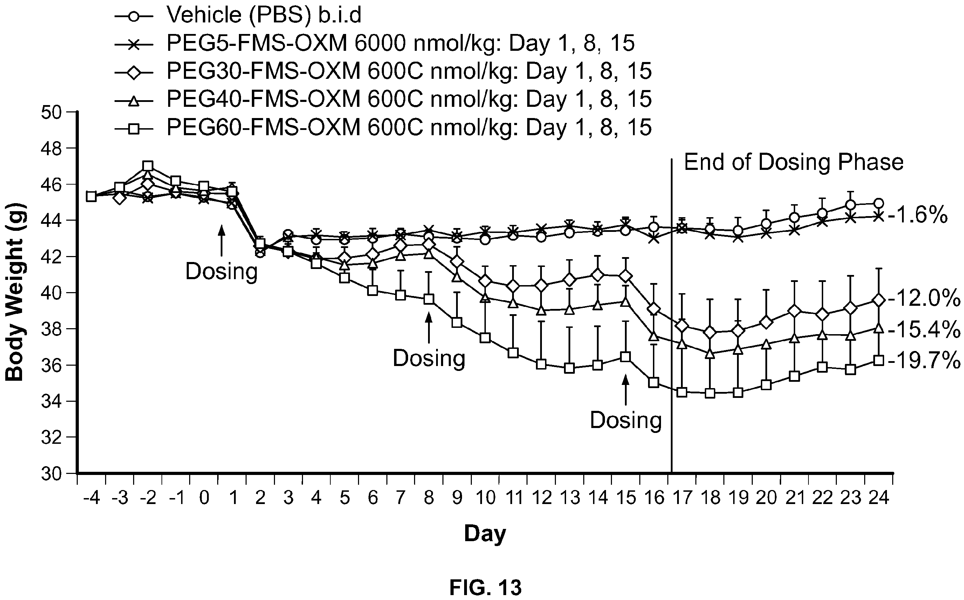

**Figure 13** shows that administration of PEG-OXM variants PEG5-FMS-OXM, PEG30-FMS-OXM, PEG40-FMS-OXM and PEG60-FMS-OXM produced marked and significant reductions in body weight when compared to controls.

### DETAILED DESCRIPTION OF THE INVENTION

The present invention provides a compound of formula (X)ₙ-Y, wherein Y is dual GLP-1/Glucagon receptor agonist consisting of an oxyntomodulin (OXM) peptide having the amino acid sequence of SEQ ID NO: 1 and bearing a free amino or hydroxyl group, and wherein X is a radical of formula (i), wherein R₁ is a polyethylene glycol (PEG) moiety; R₂ is either hydrogen or is -SO₃H at position 2 of the fluorene ring; R₃ and R₄ are each hydrogen; A is -OCO-, wherein the radical of formula (i) is linked to an amino or hydroxyl group of the drug Y; and n is an integer of at least one.

In one embodiment, the long-acting dual GLP-1/Glucagon receptor agonist consists of oxyntomodulin, polyethylene glycol polymer (PEG polymer) and 9-fluorenylmethoxycarbonyl (Fmoc) or 2-sulfo-9-fluorenylmethoxycarbonyl (FMS). In another embodiment, the composition consists of oxyntomodulin, polyethylene glycol polymer (PEG polymer) and 9-fluorenylmethoxycarbonyl (Fmoc) or 2-sulfo-9-fluorenylmethoxycarbonyl (FMS) and a pharmaceutical acceptable carrier.

In one embodiment, the terms dual "GLP-1/Glucagon receptor agonist" and "agonist" are used interchangeably herein.

In one embodiment, the term "functional" refers to the ability of the agonist or OXM provided herein to have biological activity, which include reducing weight, increasing insulin sensitivity, etc., as further provided herein.

In another embodiment, a long-acting dual GLP-1/Glucagon receptor agonist is a pegylated oxyntomodulin. In another embodiment, a long-acting dual GLP-1/Glucagon receptor agonist is a reversed pegylated oxyntomodulin. In another embodiment, a long-acting oxyntomodulin is a pegylated oxyntomodulin. In another embodiment, a long-acting oxyntomodulin is a reversed pegylated oxyntomodulin. In another embodiment, the phrases "long-acting oxyntomodulin", "reversed pegylated oxyntomodulin", "reversible PEGylated OXM", and "a composition comprising or consisting of oxyntomodulin, polyethylene glycol polymer (PEG polymer) and 9-fluorenylmethoxycarbonyl (Fmoc) or 2-sulfo-9-fluorenylmethoxycarbonyl (FMS)" are used interchangeably. In another embodiment, a long-acting oxyntomodulin is OXM linked to PEG via Fmoc or FMS.

In another embodiment, the agonist comprises a PEG polymer conjugated to the amino terminus of an oxyntomodulin peptide via Fmoc or FMS. In another embodiment, a long-acting oxyntomodulin of the invention comprises a PEG polymer conjugated to the amino terminus of an oxyntomodulin peptide via Fmoc or FMS.

In another embodiment, a long-acting oxyntomodulin is a composition comprising or consisting of oxyntomodulin, polyethylene glycol polymer (PEG polymer) and 9-fluorenylmethoxycarbonyl (Fmoc) or 2-sulfo-9-fluorenylmethoxycarbonyl (FMS) in a molar ratio of 1:0.2-10:0.2-10. In another embodiment, a long-acting oxyntomodulin is a composition comprising or consisting of oxyntomodulin, polyethylene glycol polymer (PEG polymer) and 9-fluorenylmethoxycarbonyl (Fmoc) or 2-sulfo-9-fluorenylmethoxycarbonyl (FMS) in a molar ratio of 1:0.5-2:0.5-2. In another embodiment, a long-acting oxyntomodulin is a composition comprising or consisting of oxyntomodulin, polyethylene glycol polymer (PEG polymer) and 9-fluorenylmethoxycarbonyl (Fmoc) or 2-sulfo-9-fluorenylmethoxycarbonyl (FMS) in a molar ratio of 1:1:1. In another embodiment, a long-acting oxyntomodulin includes a PEG polymer conjugated to the amino terminus of oxyntomodulin via Fmoc or FMS.

In one embodiment, the long-acting dual GLP-1/Glucagon receptor agonist is linked to PEG via a reversible linker such as Fmoc or FMS. In another embodiment, the long-acting oxyntomodulin is linked to PEG via a reversible linker such as Fmoc or FMS. In another embodiment, Fmoc and FMS are sensitive to bases and are removable under physiological conditions. In another embodiment, a reversible linker is a linker that is sensitive to bases and is removable under physiological conditions. In another embodiment, a reversible linker is a linker that is sensitive to bases and is removable under physiological conditions in the blood, plasma, or lymph. In another embodiment, a reversible linker is a linker that is sensitive to bases and is removable under physiological conditions in a body fluid. In another embodiment, a reversible linker is a linker that is removable in a body fluid having a basic pH. In another embodiment, a linker that is sensitive to bases is cleaved upon exposure to a basic environment thus releasing OXM from the linker and PEG.

In another embodiment, a reverse pegylated oxyntomodulin is a composition wherein OXM is linked to PEG via a reversible linker. In another embodiment, a reverse pegylated oxyntomodulin releases free OXM upon exposure to a basic environment. In another embodiment, a reverse pegylated oxyntomodulin releases free OXM upon exposure to blood or plasma. In another embodiment, a long-acting oxyntomodulin comprises PEG and oxyntomodulin that are not linked directly to each other, as in standard pegylation procedures, but rather both residues are linked to different positions of Fmoc or FMS which are highly sensitive to bases and are removable under regular physiological conditions. In another embodiment, regular physiological conditions include a physiologic environment such as the blood or plasma.

In another embodiment, the structures and the processes of making Fmoc and FMS are described in United States Patent No. 7585837.

In another embodiment, reverse pegylation renders OXM a long-acting OXM. In another embodiment, long-acting oxyntomodulin is an oxyntomodulin with an extended biological half-life.

In one embodiment, reverse pegylation provides protection against degradation of a dual GLP-1/Glucagon receptor agonist. In another embodiment, reverse pegylation provides protection against degradation of OXM. In another embodiment, reverse pegylation affects the Cₘₐₓ of OXM to reduce harmful side effects. In another embodiment, reverse pegylation extends the Tₘₐₓ of OXM. In another embodiment, reverse pegylation extends the circulatory half-life of OXM. In another embodiment, reverse pegylated OXM has improved bioavailability compared to non-modified OXM. In another embodiment, reverse pegylated OXM has improved biological activity compared to non-modified OXM. In some embodiments, reverse pegylation enhances the potency of OXM.

In other embodiments, a reverse pegylated OXM is at least equivalent to the non-modified OXM, in terms of biochemical measures. In other embodiments, a reverse pegylated OXM is at least equivalent to the non-modified OXM, in terms of pharmacological measures. In other embodiments, a reverse pegylated OXM is at least equivalent to the non-modified OXM, in terms of binding capacity (Kd). In other embodiments, a reverse pegylated OXM is at least equivalent to the non-modified OXM, in terms of absorption through the digestive system. In other embodiments, a reverse pegylated OXM is more stable during absorption through the digestive system than non-modified OXM.

In another embodiment, a reverse pegylated dual GLP-1/Glucagon receptor agonist exhibits improved blood area under the curve (AUC) levels compared to free agonist. In another embodiment, a reverse pegylated OXM exhibits improved blood area under the curve (AUC) levels compared to free OXM. In another embodiment, a reverse pegylated OXM exhibits improved biological activity and blood area under the curve (AUC) levels compared to free OXM. In another embodiment, a reverse pegylated dual GLP-1/Glucagon receptor agonist exhibits improved blood retention time (t_{1/2}) compared to free OXM. In another embodiment, a reverse pegylated OXM exhibits improved blood retention time (t_{1/2}) compared to free OXM. In another embodiment, a reverse pegylated OXM exhibits improved biological activity and blood retention time (t_{1/2}) compared to free OXM. In another embodiment, a reverse pegylated OXM exhibits improved blood Cₘₐₓ levels compared to free OXM, thereby reducing potentially harmful side effects. In another embodiment, a reverse pegylated OXM exhibits improved biological activity compared to free OXM. In another embodiment, the compounds are useful in improving OXM's AUC, Cₘₐₓ, t_{1/2}, biological activity, or any combination thereof. Hence, in one embodiment, the compounds are useful in improving the area under the curve (AUC) of oxyntomodulin.

The GLP-1 or glucagon agonist activity of any given glucagon analogue peptide may be quantified by determining an EC₅₀ value for that peptide in a selected assay for GLP-1 or glucagon activity. As the skilled person will be well aware, the EC50 value is a measure of the concentration at which half of that compound's maximal activity in the particular assay is achieved. In this specification, the EC₅₀ value in an assay for GLP-1 or glucagon agonist activity will be referred to as EC₅₀[GLP-1] and EC₅₀[Glu] respectively. Where EC₅₀ values for different compounds are compared, it will be understood that they describe the activity of the relevant compounds in the same assay under otherwise identical conditions.

The ratio EC₅₀[Glu]/EC₅₀[GLP-1] for the glucagon analogue peptide may be greater than the ratio EC₅₀[Glu]/EC₅₀[GLP-1] for glucagon. This may be interpreted to mean that the glucagon analogue peptide has a greater selectivity for GLP-1 receptor than glucagon.

In another embodiment, improvement of OXM's AUC, Cₘₐₓ, t_{1/2}, biological activity, or any combination thereof by conjugating a polyethylene glycol polymer (PEG polymer) to the amino terminus of free OXM via 9-fluorenylmethoxycarbonyl (Fmoc) or 2-sulfo-9-fluorenylmethoxycarbonyl (FMS) enables the reduction in dosing frequency of OXM. In another embodiment, conjugating a polyethylene glycol polymer (PEG polymer) to the amino terminus or lysine residues of OXM via 9-fluorenylmethoxycarbonyl (Fmoc) or 2-sulfo-9-fluorenylmethoxycarbonyl (FMS) enables a reduction in the dosing frequency of OXM. In another embodiment, reverse pegylation of OXM is advantageous in permitting lower dosages to be used.

SEQ ID NO: 1 has the following amino acid (AA) sequence: HSQGTFTSDYSKYLDSRRAQDFVQWLMNTKRNRNNIA (SEQ ID NO: 1).

In another embodiment, OXM is human OXM. In another embodiment, OXM is also referred to as glucagon-37 or bioactive enteroglucagon. In another embodiment, OXM is a dual GLP-1/Glucagon receptor agonist.

In one embodiment, the dual GLP-1/Glucagon receptor agonist can be chemically modified. In another embodiment, the OXM can be chemically modified. In particular, the amino acid side chains, the amino terminus and/or the carboxy acid terminus of OXM can be modified. For example, the OXM can undergo one or more of alkylation, disulphide formation, metal complexation, acylation, esterification, amidation, nitration, treatment with acid, treatment with base, oxidation or reduction. Methods for carrying out these processes are well known in the art. In particular the OXM is provided as a lower alkyl ester, a lower alkyl amide, a lower dialkyl amide, an acid addition salt, a carboxylate salt or an alkali addition salt thereof. In particular, the amino or carboxylic termini of the OXM may be derivatised by for example, esterification, amidation, acylation, oxidation or reduction. In particular, the carboxylic terminus of the OXM can be derivatised to form an amide moiety.

In one embodiment, the long-acting dual GLP-1/Glucagon receptor agonist of the invention maintains the biological activity of the unmodified agonist. In another embodiment, the OXM of the invention maintains the biological activity of unmodified OXM. In one embodiment, the long-acting OXM of the invention maintains the biological activity of unmodified OXM. In another embodiment, the long-acting OXM of the invention comprising OXM biological activity. In another embodiment, the biological activity of a long-acting OXM of the invention comprises reducing digestive secretions. In another embodiment, the biological activity of a long-acting OXM of the invention comprises reducing and delaying gastric emptying. In another embodiment, the biological activity of a long-acting OXM of the invention comprises the inhibition of the fed motility pattern in the small intestine. In another embodiment, the biological activity of a long-acting OXM of the invention comprises the inhibition of acid secretion stimulated by pentagastrin. In another embodiment, the biological activity of a long-acting OXM of the invention comprises an increase of gastric somatostatin release. In another embodiment, the biological activity of a long-acting OXM of the invention comprises potentiating the effects of peptide YY. In another embodiment, the biological activity of a long-acting OXM of the invention comprises the inhibition of ghrelin release. In another embodiment, the biological activity of long-acting OXM of the invention comprises the up-regulation of adiponectin. In another embodiment, the biological activity of long-acting OXM of the invention comprises reducing free fatty acids.. In another embodiment, the biological activity of long-acting OXM of the invention comprises reducing triglycerides. In another embodiment, the biological activity of long-acting OXM of the invention comprises reducing cholesterol. In another embodiment, the biological activity of a long-acting OXM of the invention comprises the stimulation of aminopyrine accumulation and cAMP production. In another embodiment, the biological activity of a long-acting OXM of the invention comprises binding the GLP-1 receptor or the glucagon receptor. In another embodiment, the biological activity of a long-acting OXM of the invention comprises stimulating H+ production by activating the adenylate cyclase. In another embodiment, the biological activity of a long-acting OXM of the invention comprises inhibiting histamine-stimulated gastric acid secretion. In another embodiment, the biological activity of a long-acting OXM of the invention comprises inhibiting food intake. In another embodiment, the biological activity of a long-acting OXM of the invention comprises stimulating insulin release. In another embodiment, the biological activity of a long-acting OXM of the invention comprises inhibiting exocrine pancreatic secretion. In another embodiment, the biological activity of a long-acting OXM of the invention comprises increasing insulin sensitivity. In another embodiment, the biological activity of a long-acting OXM of the invention comprises reducing glucose levels.

In one embodiment, the terms "reducing the level of" refers to a reduction of about 1-10% relative to an original, wild-type, normal or control level. In another embodiment, the reduction is of about 11-20%. In another embodiment, the reduction is of about 21-30%. In another embodiment, the reduction is of about 31-40%. In another embodiment, the reduction is of about 41-50%. In another embodiment, the reduction is of about 51-60%. In another embodiment, the reduction is of about 61-70%. In another embodiment, the reduction is of about 71-80%. In another embodiment, the reduction is of about 81-90%. In another embodiment, the reduction is of about 91-95%. In another embodiment, the reduction is of about 96-100%.

In one embodiment, the terms "increasing the level of" or "extending" refers to an increase of about 1-10% relative to an original, wild-type, normal or control level. In another embodiment, the increase is of about 11-20%. In another embodiment, the increase is of about 21-30%. In another embodiment, the increase is of about 31-40%. In another embodiment, the increase is of about 41-50%. In another embodiment, the increase is of about 51-60%. In another embodiment, the increase is of about 61-70%. In another embodiment, the increase is of about 71-80%. In another embodiment, the increase is of about 81-90%. In another embodiment, the increase is of about 91-95%. In another embodiment, the increase is of about 96-100%.

The present invention further provides the compound or a pharmaceutical composition thereof for use in inducing glucose tolerance, improving glycemic control, or both in a subject in need thereof.

The present invention further provides a method for extending the biological half-life of oxyntomodulin, consisting of the step of conjugating oxyntomodulin, a polyethylene glycol polymer (PEG polymer) and 9-fluorenylmethoxycarbonyl (Fmoc) or 2-sulfo-9-fluorenylmethoxycarbonyl (FMS) in a molar ratio of about 1:1:0.5 to about 1:1:3.5 to form a compound of the invention.

The present invention provides a compound or pharmaceutical composition of the invention for use as a medicament.

The present invention further provides the compound or pharmaceutical composition of the invention for use in reducing food intake, reducing body weight, or both in a subject. In another embodiment, the subject is afflicted with diabetes. In another embodiment, the subject is overweight. In another embodiment, the subject is afflicted with obesity.

In one embodiment, the PEG-OXM compounds provided herein induce significant reduction of glucose level without increasing insulin level. In another embodiment, the PEG-OXM compounds provided herein unexpectedly reduce glucose levels together with the reduction of fasted insulin levels following administration of a single dose of the PEG-OXM compounds (see Example 7, herein). Hence, in another embodiment, the compound of the present invention increases insulin sensitivity in a subject. In another embodiment, the agonist is conjugated to said polyethylene glycol polymer (PEG polymer) via a linker. In another embodiment, the linker is a flexible linker. In another embodiment, the linker is a non-cleavable linker.

In another embodiment, the biological activity of a long-acting dual GLP-1/Glucagon receptor agonist of the invention comprises inhibiting pancreatic secretion through a vagal neural indirect mechanism. In another embodiment, the biological activity of a long-acting dual GLP-1/Glucagon receptor agonist of the invention comprises reducing hydromineral transport through the small intestine. In another embodiment, the biological activity of a long-acting dual GLP-1/Glucagon receptor agonist of the invention comprises stimulating glucose uptake. In another embodiment, the biological activity of a long-acting dual GLP-1/Glucagon receptor agonist of the invention comprises controlling/stimulating somatostatin secretion. In another embodiment, the biological activity of a long-acting dual GLP-1/Glucagon receptor agonist of the invention comprises reduction in both food intake and body weight gain. In another embodiment, the biological activity of a long-acting dual GLP-1/Glucagon receptor agonist of the invention comprises reduction in adiposity. In another embodiment, the biological activity of a long-acting dual GLP-1/Glucagon receptor agonist of the invention comprises appetite suppression. In another embodiment, the biological activity of a long-acting dual GLP-1/Glucagon receptor agonist of the invention comprises induction of anorexia. In another embodiment, the biological activity of a long-acting dual GLP-1/Glucagon receptor agonist of the invention comprises reducing body weight in overweight and obese subjects. In another embodiment, the biological activity of a long-acting dual GLP-1/Glucagon receptor agonist of the invention comprises inducing changes in the levels of the adipose hormones leptin and adiponectin. In another embodiment, the biological activity of a long-acting dual GLP-1/Glucagon receptor agonist of the invention comprises increasing energy expenditure in addition to decreasing energy intake in overweight and obese subjects. In another embodiment, the biological activity of a long-acting dual GLP-1/Glucagon receptor agonist of the invention comprises decreasing plasma triglycerides and increased ketone bodies.

In one embodiment, the biological activity of a long-acting dual GLP-1/Glucagon receptor agonist of the invention, following acute treatment comprises decreasing plasma triglycerides and increased ketone bodies. In another embodiment, the biological activity of a long-acting dual GLP-1/Glucagon receptor agonist of the invention, following acute treatment comprises increasing expression of the gluconeogenic genes Pck1, Pgc1α, and Pdha1. In another embodiment, the biological activity of a long-acting dual GLP-1/Glucagon receptor agonist of the invention, following acute treatment comprises decreasing liver pools of acetyl-CoA, the main product of pyruvate decarboxylation, and malonyl-CoA. In another embodiment, the biological activity of a long-acting dual GLP-1/Glucagon receptor agonist of the invention, following acute treatment comprises upregulating genes that induce fatty acid oxidation (FAO) in the liver, including *Fgf21* and *Cpt1a.* In another embodiment, the biological activity of a long-acting dual GLP-1/Glucagon receptor agonist of the invention, following acute treatment comprises downregulating lipogenic genes such as *ChREBP.* In another embodiment, the biological activity of a long-acting dual GLP-1/Glucagon receptor agonist of the invention, following acute treatment comprises upregulating *Ldlr* gene.

In one embodiment, the biological activity of a long-acting dual GLP-1/Glucagon receptor agonist of the invention, following chronic treatment comprises decreasing leptin levels. In another embodiment, the biological activity of a long-acting dual GLP-1/Glucagon receptor agonist of the invention, following chronic treatment comprises increasing b-hydroxybutyrate levels.

In another embodiment, a PEG polymer is attached to the amino terminus or lysine residue of oxyntomodulin via Fmoc or FMS. In another embodiment, the terms "attached" and "linked" are used interchangeably. In another embodiment, the PEG polymer is linked to the α-amino side chain of OXM. In another embodiment, the PEG polymer is linked to the ε-amino side chain of OXM. In another embodiment, the PEG polymer is linked to one or more ε-amino side chain of OXM. In another embodiment, the PEG polymer comprises a sulfhydryl moiety.

In another embodiment, PEG is linear. In another embodiment, PEG is branched. In another embodiment, PEG has a molecular weight in the range of 200 to 200,000 Da. In another embodiment, PEG has a molecular weight in the range of 5,000 to 80,000 Da. In another embodiment, PEG has a molecular weight in the range of 5,000 to 40,000 Da. In another embodiment, PEG has a molecular weight in the range of 20,000 Da to 40,000 Da.

In another embodiment, a long-acting OXM is prepared using PEGylating agents, meaning any PEG derivative which is capable of reacting with a functional group such as NH₂, OH, SH, COOH, CHO, --N=C=O, --N=C=S, --SO₂Cl, --SO₂CH=CH₂, --PO₂Cl,-(CH₂)xHal, present at the fluorene ring of the Fmoc or FMS moiety. In another embodiment, the PEGylating agent is usually used in its mono-methoxylated form where only one hydroxyl group at one terminus of the PEG molecule is available for conjugation. In another embodiment, a bifunctional form of PEG where both termini are available for conjugation may be used if, for example, it is desired to obtain a conjugate with two peptide or protein residues covalently attached to a single PEG moiety.

In another embodiment, branched PEGs are represented as R(PEG-OH)ₘ in which R represents a central core moiety such as pentaerythritol or glycerol, and m represents the number of branching arms. The number of branching arms (m) can range from three to a hundred or more. In another embodiment, the hydroxyl groups are subject to chemical modification. In another embodiment, branched PEG molecules are described in U.S. Pat. No. 6,113,906, No. 5,919,455, No. 5,643,575, and No. 5,681,567.

In another embodiment, the PEG moiety is not attached directly to the OXM, as in the standard pegylation procedure, but rather the PEG moiety is attached through a linker such as Fmoc or FMS. In another embodiment, the linker is highly sensitive to bases and is removable under mild basic conditions. In another embodiment, OXM connected to PEG via Fmoc or FMS is equivalently active to the free OXM. In another embodiment, OXM connected to PEG via Fmoc or FMS is more active than the free OXM. In another embodiment, OXM connected to PEG via Fmoc or FMS comprises different activity than the free OXM. In another embodiment, OXM connected to PEG via Fmoc or FMS unlike the free OXM, has central nervous system activity. In another embodiment, reversible Pegylated OXM crosses the blood-brain barrier and acts on the hypothalamus to exert the biological activities provided herein. In another embodiment, OXM connected to PEG via Fmoc or FMS unlike the free OXM, cannot enter the brain through the blood brain barrier. In another embodiment, OXM connected to PEG via Fmoc or FMS comprises extended circulation half-life compared to the free OXM. In another embodiment, OXM connected to PEG via Fmoc or FMS loses its PEG moiety together with the Fmoc or FMS moiety thus recovering the free OXM.

In another embodiment, R₂, R₃ and R₄ are each hydrogen and A is --OCO--, namely the 9-fluorenylmethoxycarbonyl radical (hereinafter "Fmoc"). In another embodiment, R isSOsH at position 2 of the fluorene ring, R₃ and R₄ are each hydrogen, and A is --OCO--, namely the 2-sulfo-9-fluorenylmethoxycarbonyl radical (hereinafter "FMS").

In another embodiment, pegylation of OXM and preparation of the (PEG-Fmoc)n-OXM or (PEG-FMS)n-OXM conjugates includes attaching MAL-FMS-NHS or MAL-Fmoc-NHS to the amine component of OXM, thus obtaining a MAL-FMS-OXM or MAL-Fmoc-OXM conjugate, and then substituting PEG-SH for the maleimide moiety, producing the (PEG-FMS)n-OXM or (PEG-Fmoc)n-OXM conjugate, respectively.

In another embodiment, pegylation of OXM includes reacting MAL-FMS-NHS or MAL-Fmoc-NHS with PEG-SH, thus forming a PEG-FMS-NHS or PEG-Fmoc-NHS conjugate, and then reacting it with the amine component of OXM resulting in the desired (PEG-FMS)n-OXM or (PEG-Fmoc)n-OXM conjugate, respectively. In another embodiment, pegylation of peptides/proteins such as OXM are described in United States Patent No. 7585837. In another embodiment, reverse-pegylation of peptides/proteins such as OXM with Fmoc or FMS are described in United States Patent No. 7585837.

In another embodiment, the phrases "long acting OXM" and "reverse pegylated OXM" are used interchangeably. In another embodiment, reverse pegylated OXM is composed of PEG-FMS-OXM and PEG-Fmoc-OXM herein identified by the formulas: (PEG-FMS)n-OXM or (PEG-Fmoc)n-OXM, wherein n is an integer of at least one, and OXM is linked to the FMS or Fmoc radical through at least one amino group.

In another embodiment, surprisingly, the long acting OXM described herein is both active in its pegylated form and in its peripheral form. In another embodiment, surprisingly, the construction of (PEG-FMS)n-OXM or (PEG-Fmoc)n-OXM does not render this conjugate inactive. In another embodiment, surprisingly, the construction of (PEG-FMS)n-OXM or (PEG-Fmoc)n-OXM does not render the OXM inactive.

### Therapeutic Uses

In another embodiment, the present invention provides PEG-Fmoc-OXM and PEG-FMS-OXM and pharmaceutical compositions comprising them for use in the prevention of hyperglycemia, for improving glycemic control, for treatment of diabetes mellitus selected from non-insulin dependent diabetes mellitus (in one embodiment, Type 2 diabetes), insulin-dependent diabetes mellitus (in one embodiment, Type 1 diabetes), or gestational diabetes mellitus, or any combination thereof. In another embodiment, PEG-Fmoc-OXM and PEG-FMS-OXM and pharmaceutical compositions comprising them are utilized for treating Type 2 Diabetes. In another embodiment, PEG-Fmoc-OXM and PEG-FMS-OXM and pharmaceutical compositions comprising them are utilized for increasing sensitivity to insulin. In another embodiment, PEG-Fmoc-OXM and PEG-FMS-OXM and pharmaceutical compositions comprising them are utilized for reducing insulin resistance.

In another embodiment, PEG-Fmoc-OXM and PEG-FMS-OXM and pharmaceutical compositions comprising them may be utilized for the suppression of appetite. In another embodiment, PEG-Fmoc-OXM and PEG-FMS-OXM and pharmaceutical compositions comprising them may be utilized for inducing satiety. In another embodiment, PEG-Fmoc-OXM and PEG-FMS-OXM and pharmaceutical compositions comprising them may be utilized for the reduction of body weight. In another embodiment, PEG-Fmoc-OXM and PEG-FMS-OXM and pharmaceutical compositions comprising them may be utilized for the reduction of body fat. In another embodiment, PEG-Fmoc-OXM and PEG-FMS-OXM and pharmaceutical compositions comprising them may be utilized for the reduction of body mass index. In another embodiment, PEG-Fmoc-OXM and PEG-FMS-OXM and pharmaceutical compositions comprising them may be utilized for the reduction of food consumption. In another embodiment, PEG-Fmoc-OXM and PEG-FMS-OXM and pharmaceutical compositions comprising them may be utilized for treating obesity. In another embodiment, PEG-Fmoc-OXM and PEG-FMS-OXM and pharmaceutical compositions comprising them may be utilized for treating diabetes mellitus associated with obesity. In another embodiment, PEG-Fmoc-OXM and PEG-FMS-OXM and pharmaceutical compositions comprising them may be utilized for increasing heart rate. In another embodiment, PEG-Fmoc-OXM and PEG-FMS-OXM and pharmaceutical compositions comprising them may be utilized for increasing the basal metabolic rate (BMR). In another embodiment, PEG-Fmoc-oxyntomodulin and PEG-FMS-oxyntomodulin and pharmaceutical compositions comprising them may be utilized for increasing energy expenditure. In another embodiment, PEG-Fmoc-oxyntomodulin and PEG-FMS-oxyntomodulin and pharmaceutical compositions comprising them may be utilized for inducing glucose tolerance. In another embodiment, PEG-Fmoc-oxyntomodulin and PEG-FMS-oxyntomodulin and pharmaceutical compositions comprising them may be utilized for inducing glycemic control. In one embodiment, glycemic control refers to non-high and/or non-fluctuating blood glucose levels and/or non-high and/or non-fluctuating glycosylated hemoglobin levels.

In another embodiment, PEG-Fmoc-OXM and PEG-FMS-OXM and pharmaceutical compositions comprising them may be utilized for inhibiting weight increase. In another embodiment, PEG-Fmoc-OXM and PEG-FMS-OXM and pharmaceutical compositions comprising them may be utilized for reducing blood glucose levels (Figures 4A and 9). In another embodiment, PEG-Fmoc-OXM and PEG-FMS-OXM and pharmaceutical compositions comprising them may be utilized for decreasing caloric intake. In another embodiment, PEG-Fmoc-OXM and PEG-FMS-OXM and pharmaceutical compositions comprising them may be utilized for decreasing appetite. In another embodiment, PEG-Fmoc-OXM and PEG-FMS-OXM and pharmaceutical compositions comprising them may be utilized for weight control. In another embodiment, PEG-Fmoc-OXM and PEG-FMS-OXM and pharmaceutical compositions comprising them may be utilized for inducing or promoting weight loss. In another embodiment, PEG-Fmoc-OXM and PEG-FMS-OXM and pharmaceutical compositions comprising them may be utilized for maintaining any one or more of a desired body weight, a desired Body Mass Index, a desired appearance and good health. In another embodiment, PEG-Fmoc-OXM and PEG-FMS-OXM and pharmaceutical compositions comprising them may be utilized for controlling a lipid profile. In another embodiment, PEG-Fmoc-OXM and PEG-FMS-OXM and pharmaceutical compositions comprising them may be utilized for reducing triglyceride levels. In another embodiment, PEG-Fmoc-OXM and PEG-FMS-OXM and pharmaceutical compositions comprising them may be utilized for reducing glycerol levels (Figure 9D).

In another embodiment, PEG-Fmoc-OXM and PEG-FMS-OXM and pharmaceutical compositions comprising them may be utilized for reducing cholesterol levels. In one embodiment, the reduction in cholesterol levels is greater than the reduction observed after administration of native OXM (Figure 9C). In one embodiment, PEG-Fmoc-OXM and PEG-FMS-OXM and pharmaceutical compositions comprising them lower cholesterol levels by 60-70%. In another embodiment, PEG-Fmoc-OXM and PEG-FMS-OXM and pharmaceutical compositions comprising them lower cholesterol levels by 50-100%. In another embodiment, PEG-Fmoc-OXM and PEG-FMS-OXM and pharmaceutical compositions comprising them lower cholesterol levels by 25-90%. In another embodiment, PEG-Fmoc-OXM and PEG-FMS-OXM and pharmaceutical compositions comprising them lower cholesterol levels by 50-80%. In another embodiment, PEG-Fmoc-OXM and PEG-FMS-OXM and pharmaceutical compositions comprising them lower cholesterol levels by 40-90%. In another embodiment, PEG-Fmoc-OXM and PEG-FMS-OXM and pharmaceutical compositions comprising them are utilized for increasing HDL cholesterol levels.

In one embodiment, PEG-Fmoc-OXM and PEG-FMS-OXM and pharmaceutical compositions comprising them may be used for the purposes described herein without a significant decrease in effectiveness over the course of administration (Figures 5A, 6A, and 7). In one embodiment, PEG-Fmoc-OXM and PEG-FMS-OXM and pharmaceutical compositions comprising them remains effective for 1 day. In another embodiment, PEG-Fmoc-OXM and PEG-FMS-OXM and pharmaceutical compositions comprising them remains effective for 2-6 days. In another embodiment, PEG-Fmoc-OXM and PEG-FMS-OXM and pharmaceutical compositions comprising them remains effective for 1 week. In another embodiment, PEG-Fmoc-OXM and PEG-FMS-OXM and pharmaceutical compositions comprising them remains effective for 2 weeks. In another embodiment, PEG-Fmoc-OXM and PEG-FMS-OXM and pharmaceutical compositions comprising them remains effective for 3 weeks. In another embodiment, PEG-Fmoc-OXM and PEG-FMS-OXM and pharmaceutical compositions comprising them remains effective for 4 weeks. In another embodiment, PEG-Fmoc-OXM and PEG-FMS-OXM and pharmaceutical compositions comprising them remains effective for 6 weeks. In another embodiment, PEG-Fmoc-OXM and PEG-FMS-OXM and pharmaceutical compositions comprising them remains effective for 2 months. In another embodiment, PEG-Fmoc-OXM and PEG-FMS-OXM and pharmaceutical compositions comprising them remains effective for 4 months. In another embodiment, PEG-Fmoc-OXM and PEG-FMS-OXM and pharmaceutical compositions comprising them remains effective for 6 months. In another embodiment, PEG-Fmoc-OXM and PEG-FMS-OXM and pharmaceutical compositions comprising them remains effective for 1 year or more.

In one embodiment, PEG-Fmoc-OXM and PEG-FMS-OXM and pharmaceutical compositions comprising them may be used for the purposes described herein and may be effective immediately upon administration of the first dose (Figure 8A). In another embodiment, PEG-Fmoc-OXM and PEG-FMS-OXM and pharmaceutical compositions comprising them are effective after two or more doses have been administered.

In another embodiment, PEG-Fmoc-OXM and PEG-FMS-OXM and pharmaceutical compositions comprising them as described hereinabove may be applied to a human subject afflicted with a disease or condition that can be alleviated, inhibited, and/or treated by OXM.

In one embodiment, the OXM of the present invention may be utilized in therapeutics which requires OXM to be in a soluble form. In another embodiment, OXM of the present invention includes one or more polar amino acid, including serine and threonine which are capable of increasing protein solubility due to their hydroxyl-containing side chain.

In one embodiment, OXM of present invention is biochemically synthesized such as by using standard solid phase techniques. In another embodiment, these biochemical methods include exclusive solid phase synthesis, partial solid phase synthesis, fragment condensation, or classical solution synthesis.

In one embodiment, solid phase OXM synthesis procedures are well known to one skilled in the art and further described by John Morrow Stewart and Janis Dillaha Young, Solid Phase Protein Syntheses (2nd Ed., Pierce Chemical Company, 1984). In another embodiment, synthetic proteins are purified by preparative high performance liquid chromatography [Creighton T. (1983) Proteins, structures and molecular principles. WH Freeman and Co. N.Y.] and the composition of which can be confirmed via amino acid sequencing by methods known to one skilled in the art.

In another embodiment, recombinant protein techniques are used to generate the OXM of the present invention. In some embodiments, recombinant protein techniques are used for the generation of large amounts of the OXM. In another embodiment, recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

In another embodiment, OXM of the present invention is synthesized using a polynucleotide encoding OXM. In some embodiments, the polynucleotide encoding OXM is ligated into an expression vector, comprising a transcriptional control of a cis-regulatory sequence (e.g., promoter sequence). In some embodiments, the cis-regulatory sequence is suitable for directing constitutive expression of the OXM of the present invention.

In one embodiment, the phrase "a polynucleotide" refers to a single or double stranded nucleic acid sequence which be isolated and provided in the form of an RNA sequence, a complementary polynucleotide sequence (cDNA), a genomic polynucleotide sequence and/or a composite polynucleotide sequence (e.g., a combination of the above).

In one embodiment, "complementary polynucleotide sequence" refers to a sequence, which results from reverse transcription of messenger RNA using a reverse transcriptase or any other RNA dependent DNA polymerase. In one embodiment, the sequence can be subsequently amplified *in vivo* or *in vitro* using a DNA polymerase.

In one embodiment, "genomic polynucleotide sequence" refers to a sequence derived (isolated) from a chromosome and thus it represents a contiguous portion of a chromosome.

In one embodiment, "composite polynucleotide sequence" refers to a sequence which is at least partially complementary and at least partially genomic. In one embodiment, a composite sequence can include some exonal sequences required to encode the peptide of the present invention, as well as some intronic sequences interposing there between. In one embodiment, the intronic sequences can be of any source, including of other genes, and typically will include conserved splicing signal sequences. In one embodiment, intronic sequences include cis acting expression regulatory elements.

In one embodiment, polynucleotides are prepared using PCR techniques, or any other method or procedure known to one skilled in the art. In some embodiments, the procedure involves the ligation of two different DNA sequences (See, for example, "Current Protocols in Molecular Biology", eds. Ausubel et al., John Wiley & Sons, 1992).

A variety of prokaryotic or eukaryotic cells can be used as host-expression systems to express the OXM of the present invention. In another embodiment, these include microorganisms, such as bacteria transformed with a recombinant bacteriophage DNA, plasmid DNA or cosmid DNA expression vector containing the protein coding sequence; yeast transformed with recombinant yeast expression vectors containing the protein coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the protein coding sequence.

In one embodiment, non-bacterial expression systems are used (e.g. mammalian expression systems such as CHO cells) to express the OXM of the present invention. In one embodiment, the expression vector used to express polynucleotides of the present invention in mammalian cells is pCI-DHFR vector comprising a CMV promoter and a neomycin resistance gene.

In another embodiment, in bacterial systems, a number of expression vectors can be advantageously selected depending upon the use intended for the protein expressed. In one embodiment, large quantities of OXM are desired. In one embodiment, vectors that direct the expression of high levels of the protein product, possibly as a fusion with a hydrophobic signal sequence, which directs the expressed product into the periplasm of the bacteria or the culture medium where the protein product is readily purified are desired. In one embodiment, certain fusion protein engineered with a specific cleavage site to aid in recovery of the protein. In one embodiment, vectors adaptable to such manipulation include the pET series of *E. coli* expression vectors [Studier et al., Methods in Enzymol. 185:60-89 (1990)].

In one embodiment, yeast expression systems are used. In one embodiment, a number of vectors containing constitutive or inducible promoters can be used in yeast as disclosed in U.S. Pat. Application. No: 5,932,447. In another embodiment, vectors which promote integration of foreign DNA sequences into the yeast chromosome are used.

In one embodiment, the expression vector can further include additional polynucleotide sequences that allow, for example, the translation of several proteins from a single mRNA such as an internal ribosome entry site (IRES) and sequences for genomic integration of the promoter-chimeric protein.

In one embodiment, mammalian expression vectors include pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

In another embodiment, expression vectors containing regulatory elements from eukaryotic viruses such as retroviruses are used by the present invention. SV40 vectors include pSVT7 and pMT2. In another embodiment, vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In one embodiment, plant expression vectors are used. In one embodiment, the expression of the dual GLP-1/Glucagon receptor agonist coding sequence (such as OXM) is driven by a number of promoters. In another embodiment, viral promoters such as the 35S RNA and 19S RNA promoters of CaMV [Brisson et al., Nature 310:511-514 (1984)], or the coat protein promoter to TMV [Takamatsu et al., EMBO J. 6:307-311 (1987)] are used. In another embodiment, plant promoters are used such as the small subunit of RUBISCO [Coruzzi et al., EMBO J. 3:1671-1680 (1984); and Brogli et al., Science 224:838-843 (1984)] or heat shock promoters, e.g., soybean hsp17.5-E or hsp17.3-B [Gurley et al., Mol. Cell. Biol. 6:559-565 (1986)]. In one embodiment, constructs are introduced into plant cells using Ti plasmid, Ri plasmid, plant viral vectors, direct DNA transformation, microinjection, electroporation and other techniques well known to the skilled artisan. See, for example, Weissbach & Weissbach [Methods for Plant Molecular Biology, Academic Press, NY, Section VIII, pp 421-463 (1988)]. Other expression systems such as insects and mammalian host cell systems can also be used by the present invention.

It will be appreciated that other than containing the necessary elements for the transcription and translation of the inserted coding sequence (encoding the protein), the expression construct can also include sequences engineered to optimize stability, production, purification, yield or activity of the expressed protein.

Various methods, in some embodiments, can be used to introduce the expression vector into the host cell system. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

In one embodiment, transformed cells are cultured under effective conditions, which allow for the expression of high amounts of recombinant OXM. In another embodiment, effective culture conditions include effective media, bioreactor, temperature, pH and oxygen conditions that permit protein production. In one embodiment, an effective medium refers to any medium in which a cell is cultured to produce the recombinant OXM of the present invention. In another embodiment, a medium typically includes an aqueous solution having assimilable carbon, nitrogen and phosphate sources, and appropriate salts, minerals, metals and other nutrients, such as vitamins. In one embodiment, cells of the present invention can be cultured in conventional fermentation bioreactors, shake flasks, test tubes, microtiter dishes and petri plates. In another embodiment, culturing is carried out at a temperature, pH and oxygen content appropriate for a recombinant cell. In another embodiment, culturing conditions are within the expertise of one of ordinary skill in the art.

In one embodiment, depending on the vector and host system used for production, resultant OXM of the present invention either remain within the recombinant cell, secreted into the fermentation medium, secreted into a space between two cellular membranes, such as the periplasmic space in *E. coli;* or retained on the outer surface of a cell or viral membrane.

In one embodiment, following a predetermined time in culture, recovery of the recombinant OXM is effected.

In one embodiment, the phrase "recovering the recombinant OXM" used herein refers to collecting the whole fermentation medium containing the OXM and need not imply additional steps of separation or purification.

In one embodiment, OXM of the present invention is purified using a variety of standard protein purification techniques, such as affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

In one embodiment, to facilitate recovery, the expressed coding sequence can be engineered to encode the protein of the present invention and fused cleavable moiety. In one embodiment, a fusion protein can be designed so that the protein can be readily isolated by affinity chromatography; e.g., by immobilization on a column specific for the cleavable moiety. In one embodiment, a cleavage site is engineered between the protein and the cleavable moiety and the protein can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)]. In another embodiment, the OXM of the present invention is retrieved in "substantially pure" form. In another embodiment, the phrase "substantially pure" refers to a purity that allows for the effective use of the OXM in the applications described herein.

In one embodiment, the dual GLP-1/Glucagon receptor agonist can also be synthesized using *in vitro* expression systems. In one embodiment, *in vitro* synthesis methods are well known in the art and the components of the system are commercially available.

In another embodiment, *in vitro* binding activity is ascertained by measuring the ability of native, recombinant and/or reverse pegylated dual GLP-1/Glucagon receptor agonist as described herein as well as pharmaceutical compositions comprising the same to treat or ameliorate diseases or conditions such as: diabetes mellitus, obesity, eating disorders, metabolic disorders, etc. In another embodiment, in vivo activity is deduced by known measures of the disease that is being treated.

In another embodiment, a dose of reverse pegylated OXM of the present invention comprises from 0.005 to 0.1 milligrams/kg OXM peptide. In another embodiment, a dose of reverse pegylated OXM of the present invention comprises from 0.005 to 0.5 milligrams/kg OXM peptide. In another embodiment, a dose of reverse pegylated OXM of the present invention comprises from 0.05 to 0.1 micrograms OXM peptide. In another embodiment, a dose of reverse pegylated OXM comprises from 0.005 to 0.1 milligrams/kg OXM peptide in an injectable solution.

In another embodiment, a dose of reverse pegylated OXM is administered once a day. In another embodiment, a dose of reverse pegylated OXM is administered once every 36 hours. In another embodiment, a dose of reverse pegylated OXM is administered once every 48 hours. In another embodiment, a dose of reverse pegylated OXM is administered once every 60 hours. In another embodiment, a dose of reverse pegylated OXM is administered once every 72 hours. In another embodiment, a dose of reverse pegylated OXM is administered once every 84 hours. In another embodiment, a dose of reverse pegylated OXM is administered once every 96 hours. In another embodiment, a dose of reverse pegylated OXM is administered once every 5 days. In another embodiment, a dose of reverse pegylated OXM is administered once every 6 days. In another embodiment, a dose of reverse pegylated OXM is administered once every 7 days. In another embodiment, a dose of reverse pegylated OXM is administered once every 8-10 days. In another embodiment, a dose of reverse pegylated OXM is administered once every 10-12 days. In another embodiment, a dose of reverse pegylated OXM is administered once every 12-15 days. In another embodiment, a dose of reverse pegylated OXM is administered once every 15-25 days.

In another embodiment, reverse pegylated OXM of the present invention is administered by an intramuscular (IM) injection, subcutaneous (SC) injection, or intravenous (IV) injection once a week.

In another embodiment, the reverse pegylated OXM of the present invention can be provided to the individual *per se.* In one embodiment, the reverse pegylated OXM of the present invention can be provided to the individual as part of a pharmaceutical composition where it is mixed with a pharmaceutically acceptable carrier.

In another embodiment, a "pharmaceutical composition" refers to a preparation of long-acting OXM as described herein with other chemical components such as physiologically suitable carriers and excipients. The purpose of a pharmaceutical composition is to facilitate administration of a compound to an organism. In another embodiment, a reverse pegylated OXM is accountable for the biological effect.

In another embodiment, any of the compositions of this invention will comprise at least a reverse pegylated OXM. The present invention may be used in combined preparations. A "combined preparation" defines especially a "kit of parts" in the sense that the combination partners as defined above can be dosed independently or by use of different fixed combinations with distinguished amounts of the combination partners i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In another embodiment, the phrases "physiologically acceptable carrier" and "pharmaceutically acceptable carrier" which be interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979).

In another embodiment, "excipient" refers to an inert substance added to a pharmaceutical composition to further facilitate administration of a long-acting OXN. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

In another embodiment, suitable routes of administration of the compounds of the present invention, for example, include oral, rectal, transmucosal, transnasal, intestinal or parenteral delivery, including intramuscular, subcutaneous and intramedullary injections as well as intrathecal, direct intraventricular, intravenous, intraperitoneal, intranasal, or intraocular injections.

The present invention also provides the compound or composition herein for use as a medicament. Examples of peripheral routes include oral, rectal, parenteral e.g. intravenous, intramuscular, or intraperitoneal, mucosal e.g. buccal, sublingual, nasal, subcutaneous or transdermal administration, including administration by inhalation. Preferred dose amounts of OXM for the medicaments are given below.

In an embodiment, the pharmaceutical composition comprises a reverse pegylated OXM and a pharmaceutically suitable carrier, in a form suitable for oral, rectal, parenteral, e.g. intravenous, intramuscular, or intraperitoneal, mucosal e.g. buccal, sublingual, nasal, subcutaneous or transdermal administration, including administration by inhalation. If in unit dosage form, the dose per unit may be, for example, as described below or as calculated on the basis of the per kg doses given below.

In another embodiment, the preparation is administered in a local rather than systemic manner, for example, via injection of the preparation directly into a specific region of a patient's body. In another embodiment, a reverse pegylated OXM is formulated in an intranasal dosage form. In another embodiment, a reverse pegylated OXM is formulated in an injectable dosage form.

Various embodiments of dosage ranges are contemplated by this invention: the OXM peptide component within of the reverse pegylated OXM composition is administered in a range of 0.01-0.5 milligrams/kg body weight per 3 days (only the weight of the OXM within the reverse pegylated OXM composition is provided as the size of PEG can differ substantially). In another embodiment, the OXM peptide component within of the reverse pegylated OXM composition is administered in a range of 0.01-0.5 milligrams/kg body weight per 7 days. In another embodiment, the OXM peptide component within of the reverse pegylated OXM composition is administered in a range of 0.01-0.5 milligrams/kg body weight per 10 days. In another embodiment, the OXM peptide component within of the reverse pegylated OXM composition is administered in a range of 0.01-0.5 milligrams/kg body weight per 14 days. In another embodiment, unexpectedly, the effective amount of OXM in a reverse pegylated OXM composition is 1/4-1/10 of the effective amount of free OXM. In another embodiment, unexpectedly, reverse pegylation of OXM enables limiting the amount of OXM prescribed to a patient by at least 50% compared with free OXM. In another embodiment, unexpectedly, reverse pegylation of OXM enables limiting the amount of OXM prescribed to a patient by at least 70% compared with free OXM. In another embodiment, unexpectedly, reverse pegylation of OXM enables limiting the amount of OXM prescribed to a patient by at least 75% compared with free OXM. In another embodiment, unexpectedly, reverse pegylation of OXM enables limiting the amount of OXM prescribed to a patient by at least 80% compared with free OXM. In another embodiment, unexpectedly, reverse pegylation of OXM enables limiting the amount of OXM prescribed to a patient by at least 85% compared with free OXM. In another embodiment, unexpectedly, reverse pegylation of OXM enables limiting the amount of OXM prescribed to a patient by at least 90% compared with free OXM.

In another embodiment, the OXM peptide component within of the reverse pegylated OXM composition is administered in a range of 0.01-0.5 milligrams/kg body weight once every 3 days (only the weight of the OXM within the reverse pegylated OXM composition is provided as the size of PEG can differ substantially). In another embodiment, the OXM peptide component within of the reverse pegylated OXM composition is administered in a range of 0.01-0.5 milligrams/kg body weight once every 7 days. In another embodiment, the OXM peptide component within of the reverse pegylated OXM composition is administered in a range of 0.01-0.5 milligrams/kg body weight once every 10 days. In another embodiment, the OXM peptide component within of the reverse pegylated OXM composition is administered in a range of 0.01-0.5 milligrams/kg body weight once every 14 days.

In another embodiment, reverse pegylated OXM compared to free OXM both reduces the effective dosing frequency by at least 2-fold and reduces the effective weekly dose by at least 2-fold, thus limiting the risk of adverse events and increasing compliance with the use of OXM therapy. In another embodiment, reverse pegylated OXM compared to free OXM both reduces the effective dosing frequency by at least 3-fold and reduces the effective weekly dose by at least 3-fold, thus limiting the risk of adverse events and increasing compliance with the use of OXM therapy. In another embodiment, reverse pegylated OXM compared to free OXM both reduces the effective dosing frequency by at least 4-fold and reduces the effective weekly dose by at least 4-fold, thus limiting the risk of adverse events and increasing compliance with the use of OXM therapy. In another embodiment, reverse pegylated OXM compared to free OXM both reduces the effective dosing frequency by at least 5-fold and reduces the effective weekly dose by at least 5-fold, thus limiting the risk of adverse events and increasing compliance with the use of OXM therapy. In another embodiment, reverse pegylated OXM compared to free OXM both reduces the effective dosing frequency by at least 6-fold and reduces the effective weekly dose by at least 6-fold, thus limiting the risk of adverse events and increasing compliance with the use of OXM therapy. In another embodiment, effective dosing frequency and effective weekly dose are based on: (1) the weight of administered OXM component within the reverse pegylated OXM composition; and (2) the weight of administered OXM component within the free OXM (unmodified OXM) composition.

In another embodiment, reduction in the dosing frequency of OXM is enabled by reverse pegylating OXM as described hereinabove. In another embodiment, the term compliance comprises adherence. In another embodiment, increasing the compliance of patients in need of OXM therapy includes reducing the frequency of administration of OXM. In another embodiment, reduction in the frequency of administration of the OXM is achieved thanks to reverse pegylation which render the OXM more stable and more potent. In another embodiment, reduction in the frequency of administration of the OXM is achieved as a result of increasing T1/2 of the OXM. In another embodiment, reduction in the frequency of administration of the OXM is achieved as a result of reducing blood clearance of OXM. In another embodiment, reduction in the frequency of administration of the OXM is achieved as a result of increasing T1/2 of the OXM. In another embodiment, reduction in the frequency of administration of the OXM is achieved as a result of increasing the AUC measure of the OXM.

In another embodiment, a reverse pegylated OXM is administered to a subject once a day. In another embodiment, a reverse pegylated OXM is administered to a subject once every two days. In another embodiment, a reverse pegylated OXM is administered to a subject once every three days. In another embodiment, a reverse pegylated OXM is administered to a subject once every four days. In another embodiment, a reverse pegylated OXM is administered to a subject once every five days. In another embodiment, a reverse pegylated OXM is administered to a subject once every six days. In another embodiment, a reverse pegylated OXM is administered to a subject once every week. In another embodiment, a reverse pegylated OXM is administered to a subject once every 7-14 days. In another embodiment, a reverse pegylated OXM is administered to a subject once every 10-20 days. In another embodiment, a reverse pegylated OXM is administered to a subject once every 5-15 days. In another embodiment, a reverse pegylated OXM is administered to a subject once every 15-30 days.

In another embodiment, a pegylated OXM is administered to a subject once a day. In another embodiment, a pegylated OXM is administered to a subject once every two days. In another embodiment, a pegylated OXM is administered to a subject once every three days. In another embodiment, a pegylated OXM is administered to a subject once every four days. In another embodiment, a pegylated OXM is administered to a subject once every five days. In another embodiment, a pegylated OXM is administered to a subject once every six days. In another embodiment, a pegylated OXM is administered to a subject once every week. In another embodiment, a pegylated OXM is administered to a subject once every 7-14 days. In another embodiment, a pegylated OXM is administered to a subject once every 10-20 days. In another embodiment, a pegylated OXM is administered to a subject once every 5-15 days. In another embodiment, a pegylated OXM is administered to a subject once every 15-30 days.

In one embodiment, pegylated OXM variants provided herein unexpectedly reduce glucose together with reduction of fasted insulin levels following administration of a single dose of the PEG-OXM variant. In another embodiment, the pegylated OXM variants provided herein lead to increasing the sensitivity of a subject to insulin (see Example 6).

Oral administration, in one embodiment, comprises a unit dosage form comprising tablets, capsules, lozenges, chewable tablets, suspensions, emulsions and the like. Such unit dosage forms comprise a safe and effective amount of OXM of the invention, each of which is in one embodiment, from about 0.7 or 3.5 mg to about 280 mg/70 kg, or in another embodiment, about 0.5 or 10 mg to about 210 mg/70 kg. The pharmaceutically-acceptable carriers suitable for the preparation of unit dosage forms for peroral administration are well-known in the art. In some embodiments, tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. In one embodiment, glidants such as silicon dioxide can be used to improve flow characteristics of the powder-mixture. In one embodiment, coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. In some embodiments, the selection of carrier components depends on secondary considerations like taste, cost, and shelf stability, which are not critical for the purposes of this invention, and can be readily made by a person skilled in the art.

In one embodiment, the oral dosage form comprises predefined release profile. In one embodiment, the oral dosage form of the present invention comprises an extended release tablets, capsules, lozenges or chewable tablets. In one embodiment, the oral dosage form of the present invention comprises a slow release tablets, capsules, lozenges or chewable tablets. In one embodiment, the oral dosage form of the present invention comprises an immediate release tablets, capsules, lozenges or chewable tablets. In one embodiment, the oral dosage form is formulated according to the desired release profile of the long-acting OXM as known to one skilled in the art.

In another embodiment, compositions for use according to this invention comprise solutions or emulsions, which in another embodiment are aqueous solutions or emulsions comprising a safe and effective amount of the compounds of the present invention and optionally, other compounds, intended for topical intranasal administration. In some embodiments, the compositions comprise from about 0.001% to about 10.0% w/v of a subject compound, more preferably from about 00.1% to about 2.0, which is used for systemic delivery of the compounds by the intranasal route.

In another embodiment, the pharmaceutical compositions are administered by intravenous, intra-arterial, subcutaneous or intramuscular injection of a liquid preparation. In another embodiment, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the compounds of the present invention are combined with an additional appropriate therapeutic agent or agents, prepared and applied as solutions, suspensions, or emulsions in a physiologically acceptable diluent with or without a pharmaceutical carrier.

In one embodiment, pharmaceutical compositions of the present invention are manufactured by processes well known in the art, e.g., by means of conventional mixing, dissolving, granulating, dragee-making, levigating, emulsifying, encapsulating, entrapping or lyophilizing processes.

In one embodiment, pharmaceutical compositions for use in accordance with the present invention are formulated in conventional manner using one or more physiologically acceptable carriers comprising excipients and auxiliaries, which facilitate processing of OXM into preparations which, can be used pharmaceutically. In one embodiment, formulation is dependent upon the route of administration chosen.

In one embodiment, injectables of the invention are formulated in aqueous solutions. In one embodiment, injectables of the invention are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In another embodiment, formulations for injection are presented in unit dosage form, e.g., in ampoules or in multidose containers with optionally, an added preservative. In another embodiment, compositions are suspensions, solutions or emulsions in oily or aqueous vehicles, and contain formulatory agents such as suspending, stabilizing and/or dispersing agents.

The compositions also comprise, in another embodiment, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfite and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In one embodiment, pharmaceutical compositions for parenteral administration include aqueous solutions of the active preparation in water-soluble form. Additionally, suspensions of long acting OXM, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances which increase the viscosity of the suspension, such as sodium carboxymethyl cellulose, sorbitol or dextran. In another embodiment, the suspension also contain suitable stabilizers or agents which increase the solubility of long acting OXM to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In one embodiment, long acting OXM is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In one embodiment, the preparation of the present invention is formulated in rectal compositions such as suppositories or retention enemas, using, e.g., conventional suppository bases such as cocoa butter or other glycerides.

In one embodiment, pharmaceutical compositions suitable for use in context of the present invention include compositions wherein long acting OXM is contained in an amount effective to achieve the intended purpose. In another embodiment, a therapeutically effective amount means an amount of long acting OXM effective to prevent, alleviate or ameliorate symptoms of disease or prolong the survival of the subject being treated.

In one embodiment, determination of a therapeutically effective amount is well within the capability of those skilled in the art.

The compositions may also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfite and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween^{™} brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment, the pharmaceutically-acceptable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hydroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel^{™}, RC-591), tragacanth and sodium alginate; typical wetting agents include lecithin and polyethylene oxide sorbitan (e.g. polysorbate 80). Typical preservatives include methyl paraben and sodium benzoate. In another embodiment, peroral liquid compositions also contain one or more components such as sweeteners, flavoring agents and colorants disclosed above.

The compositions also include incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in vivo* clearance.

Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors.

In another embodiment, the modified compounds exhibit substantially longer half-lives in blood following intravenous injection than do the corresponding unmodified compounds. In one embodiment, modifications also increase the compound's solubility in aqueous solution, eliminate aggregation, enhance the physical and chemical stability of the compound, and greatly reduce the immunogenicity and reactivity of the compound. In another embodiment, the desired *in vivo* biological activity is achieved by the administration of such polymer-compound abducts less frequently or in lower doses than with the unmodified compound.

In another embodiment, preparation of effective amount or dose can be estimated initially from in vitro assays. In one embodiment, a dose can be formulated in animal models and such information can be used to more accurately determine useful doses in humans.

Toxicity and therapeutic efficacy of the long acting agonist (such as OXM) as described herein can be determined by standard pharmaceutical procedures *in vitro,* in cell cultures or experimental animals. In one embodiment, the data obtained from these *in vitro* and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fing1, et al., (1975) "The Pharmacological Basis of Therapeutics", Ch. 1 p.1].

In one embodiment, depending on the severity and responsiveness of the condition to be treated, dosing can be of a single or a plurality of administrations, with course of treatment lasting from several days to several weeks or until cure is effected or diminution of the disease state is achieved.

In one embodiment, the amount of a composition to be administered will, of course, be dependent on the subject being treated, the severity of the affliction, the manner of administration, the judgment of the prescribing physician, etc.

In one embodiment, compositions including the preparation of the present invention formulated in a compatible pharmaceutical carrier are also prepared, placed in an appropriate container, and labeled for treatment of an indicated condition.

In another embodiment, a pegylated or reverse pegylated dual GLP-1/Glucagon receptor agonist as described herein is administered via systemic administration. In another embodiment, a pegylated or reverse pegylated dual GLP-1/Glucagon receptor agonist as described herein is administered by intravenous, intramuscular or subcutaneous injection. In another embodiment, a pegylated or reverse pegylated dual GLP-1/Glucagon receptor agonist as described herein is lyophilized (i.e., freeze-dried) preparation in combination with complex organic excipients and stabilizers such as nonionic surface active agents (i.e., surfactants), various sugars, organic polyols and/or human serum albumin. In another embodiment, a pharmaceutical composition comprises a lyophilized pegylated or reverse pegylated dual GLP-1/Glucagon receptor agonist as described herein in sterile water for injection. In another embodiment, a pharmaceutical composition comprises a lyophilized pegylated or reverse pegylated dual GLP-1/Glucagon receptor agonist as described herein in sterile PBS for injection. In another embodiment, a pharmaceutical composition comprises a lyophilized pegylated or reverse pegylated dual GLP-1/Glucagon receptor agonist as described herein in sterile 0.9% NaCl for injection.

In another embodiment, the pharmaceutical composition comprises a pegylated or reverse pegylated dual GLP-1/Glucagon receptor agonist as described herein and complex carriers such as human serum albumin, polyols, sugars, and anionic surface active stabilizing agents. See, for example, WO 89/10756 (Hara et al.- containing polyol and p-hydroxybenzoate). In another embodiment, the pharmaceutical composition comprises a reverse pegylated dual GLP-1/Glucagon receptor agonist as described herein and lactobionic acid and an acetate/glycine buffer. In another embodiment, the pharmaceutical composition comprises a pegylated or reverse pegylated dual GLP-1/Glucagon receptor agonist as described herein and amino acids, such as arginine or glutamate that increase the solubility of interferon compositions in water. In another embodiment, the pharmaceutical composition comprises a lyophilized pegylated or reverse pegylated dual GLP-1/Glucagon receptor agonist as described herein and glycine or human serum albumin (HSA), a buffer (e g. acetate) and an isotonic agent (e.g NaCl). In another embodiment, the pharmaceutical composition comprises a lyophilized pegylated or reverse pegylated dual GLP-1/Glucagon receptor agonist as described herein and phosphate buffer, glycine and HSA.

In another embodiment, the pharmaceutical composition comprising a pegylated or reverse pegylated dual GLP-1/Glucagon receptor agonist as described herein is stabilized when placed in buffered solutions having a pH between about 4 and 7.2. In another embodiment, the pharmaceutical composition comprising a pegylated or reverse pegylated dual GLP-1/Glucagon receptor agonist as described herein is stabilized with an amino acid as a stabilizing agent and in some cases a salt (if the amino acid does not contain a charged side chain).

In another embodiment, the pharmaceutical composition comprising a pegylated or reverse pegylated dual GLP-1/Glucagon receptor agonist as described herein is a liquid composition comprising a stabilizing agent at between about 0.3% and 5% by weight which is an amino acid.

In another embodiment, the pharmaceutical composition comprising a pegylated or reverse pegylated dual GLP-1/Glucagon receptor agonist as described herein provides dosing accuracy and product safety. In another embodiment, the pharmaceutical composition comprising a pegylated or reverse pegylated dual GLP-1/Glucagon receptor agonist as described herein provides a biologically active, stable liquid formulation for use in injectable applications. In another embodiment, the pharmaceutical composition comprises a non-lyophilized pegylated or reverse pegylated dual GLP-1/Glucagon receptor agonist as described herein.

In another embodiment, the pharmaceutical composition comprising a pegylated or reverse pegylated dual GLP-1/Glucagon receptor agonist as described herein provides a liquid formulation permitting storage for a long period of time in a liquid state facilitating storage and shipping prior to administration.

In another embodiment, the pharmaceutical composition comprising a pegylated or reverse pegylated dual GLP-1/Glucagon receptor agonist as described herein comprises solid lipids as matrix material. In another embodiment, the injectable pharmaceutical composition comprising a pegylated or reverse pegylated dual GLP-1/Glucagon receptor agonist as described herein comprises solid lipids as matrix material. In another embodiment, the production of lipid microparticles by spray congealing was described by Speiser (Speiser and al., Pharm. Res. 8 (1991) 47-54) followed by lipid nanopellets for peroral administration (Speiser EP 0167825 (1990)). In another embodiment, lipids, which are used, are well tolerated by the body (e. g. glycerides composed of fatty acids which are present in the emulsions for parenteral nutrition).

In another embodiment, the pharmaceutical composition comprising a pegylated or reverse pegylated dual GLP-1/Glucagon receptor agonist as described herein is in the form of liposomes (J. E. Diederichs and al., Pharm./nd. 56 (1994) 267- 275).

In another embodiment, the pharmaceutical composition comprising a pegylated or reverse pegylated dual GLP-1/Glucagon receptor agonist as described herein comprises polymeric microparticles. In another embodiment, the injectable pharmaceutical composition comprising a pegylated or reverse pegylated dual GLP-1/Glucagon receptor agonist as described herein comprises polymeric microparticles. In another embodiment, the pharmaceutical composition comprising a pegylated or reverse pegylated dual GLP-1/Glucagon receptor agonist as described herein comprises nanoparticles. In another embodiment, the pharmaceutical composition comprising a reverse pegylated dual GLP-1/Glucagon receptor agonist as described herein comprises liposomes. In another embodiment, the pharmaceutical composition comprising a pegylated or reverse pegylated OXM as described herein comprises lipid emulsion. In another embodiment, the pharmaceutical composition comprising a pegylated or reverse pegylated dual GLP-1/Glucagon receptor agonist as described herein comprises microspheres. In another embodiment, the pharmaceutical composition comprising a pegylated or reverse pegylated dual GLP-1/Glucagon receptor agonist as described herein comprises lipid nanoparticles. In another embodiment, the pharmaceutical composition comprising a pegylated or reverse pegylated dual GLP-1/Glucagon receptor agonist as described herein comprises lipid nanoparticles comprising amphiphilic lipids. In another embodiment, the pharmaceutical composition comprising a pegylated or reverse pegylated dual GLP-1/Glucagon receptor agonist as described herein comprises lipid nanoparticles comprising a drug, a lipid matrix and a surfactant. In another embodiment, the lipid matrix has a monoglyceride content which is at least 50% w/w.

The compositions of the present invention may be presented in a pack or dispenser device, such as an FDA approved kit, which contain one or more unit dosage forms containing the long acting dual GLP-1/Glucagon receptor agonist. The pack may comprise metal or plastic foil, such as a blister pack. The pack or dispenser device may be accompanied by instructions for administration. The pack or dispenser may be accommodated by a notice associated with the container in a form prescribed by a governmental agency regulating the manufacture, use or sale of pharmaceuticals, which notice is reflective of approval by the agency of the form of the compositions or human or veterinary administration. Such notice may be labeling approved by the U.S. Food and Drug Administration for prescription drugs or of an approved product insert.

It will be appreciated that the pegylated or reverse pegylated dual GLP-1/Glucagon receptor agonist of the present invention can be provided to the individual with additional active agents to achieve an improved therapeutic effect as compared to treatment with each agent by itself. In another embodiment, measures (e.g., dosing and selection of the complementary agent) are taken to adverse side effects which are associated with combination therapies.

Additional objects, advantages, and novel features of the present invention will become apparent to one ordinarily skilled in the art upon examination of the following examples. Additionally, each of the various embodiments and aspects of the present invention as claimed in the claims section below finds experimental support in the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

### MATERIALS AND METHODS

### PEG₄₀-Fmoc-OXM and PEG₄₀-FMS-OXM synthesis

OXM synthesis: Oxyntomodulin of sequence: HSQGTFTSDYSKYLDSRRAQDFVQWLMNTKRNRNNIA (SEQ ID NO: 1) was synthesized by the solid phase method employing the Fmoc-strategy throughout the peptide chain assembly (Almac Sciences, Scotland). The peptide sequence was assembled using the following steps: (1) Capping: the resin was capped using 0.5M acetic anhydride (Fluka) solution in DMF (Rathburn); (2) De-protection: Fmoc-protecting group was removed from the growing peptide chain using 20% v/v piperidine (Rathburn) solution in DMF (Rathburn); and (3) Amino Acid Coupling: 0.5 M Amino acid (Novabiochem) solution in DMF (Rathburn) was activated using 1M HOBt (Carbosynth) solution in DMF (Rathburn) and 1M DIC (Carbosynth) solution in DMF (Rathburn). Four equivalents of each amino acid were used per coupling.

The crude peptide was cleaved from the resin, and the protecting groups were removed by stirring in a cocktail of Triisopropylsilane (Fluka), water, dimethylsulphide (Aldrich), ammonium iodide (Aldrich) and TFA (Applied Biosystems) for 4 hours. The crude peptide was then collected by precipitation from cold diethyl ether.

Peptide Purification: Crude peptide was dissolved in acetonitrile (Rathburn)/water (MilliQ) (5:95) and loaded onto the preparative HPLC column. The chromatographic parameters are as follows: Column: Phenomenex Luna C18 250mm x 30mm, 15µm, 300A; Mobile Phase A: water + 0.1% v/v TFA (Applied Biosystems); Mobile Phase B: acetonitrile (Rathburn) + 0.1% v/v TFA (Applied Biosystems); UV Detection: 214 or 220 nm; Gradient: 25%B to 31%B over 4 column volumes; and flow rate 43mL/min.

### Stage 2 - Linker Synthesis- Synthesis of MAL-FMS-NHS Linker:

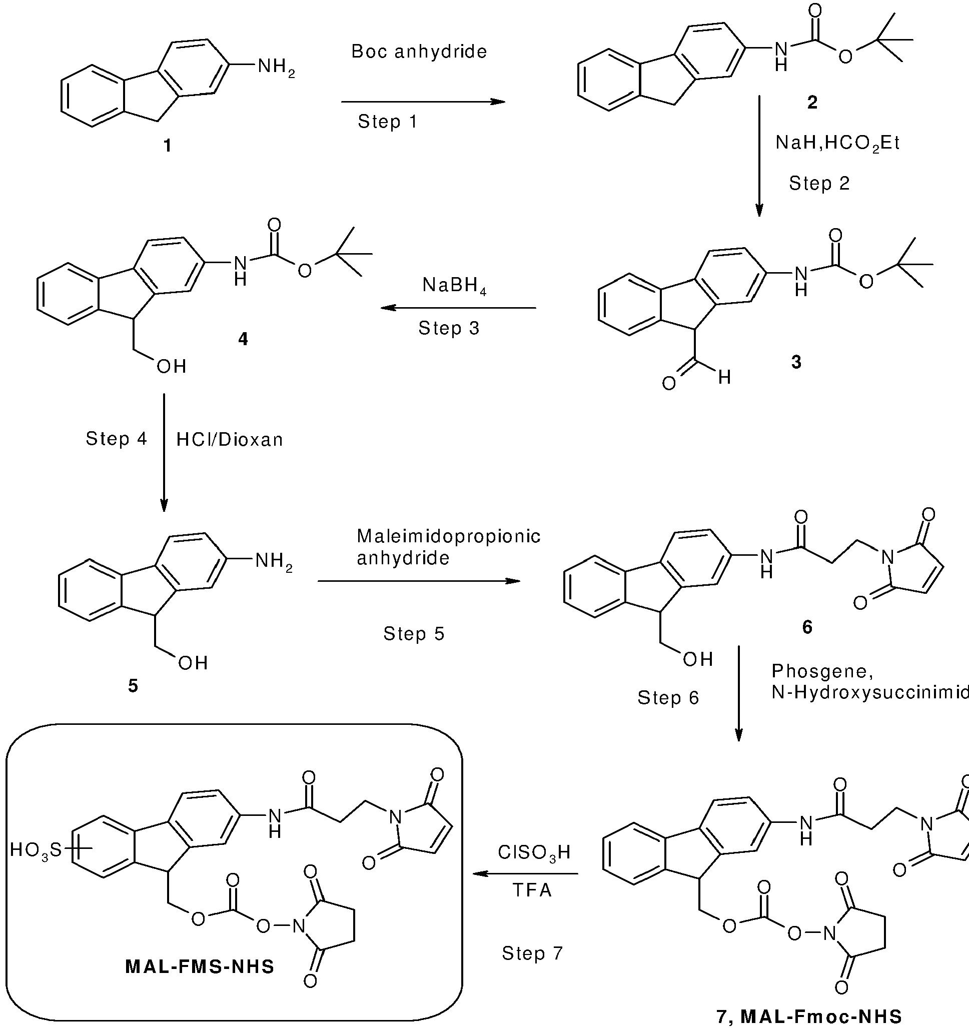

The synthesis of compounds 2-5 was based on the procedures described by Albericio et al. in Synthetic Communication, 2001, 31(2), 225-232.

2-(Boc-amino)fluorene (2): 2- Aminofluorene (18g, 99mmol) was suspended in a mixture of dioxane:water (2:1) (200ml) and 2N NaOH (60ml) in an ice bath with magnetic stirring. Boc₂O (109mmol, 1.1 eq) was then added, and stirring continued at RT. The reaction was monitored by TLC (Rf= 0.5, Hexane/ Ethyl Acetate 2:1), and the pH was maintained between 9-10 by addition of 2N NaOH. Upon reaction completion, the suspension was acidified with 1M KHSO₄ to pH=3. The solid phase was filtered and washed with cold water (50ml), dioxane-water (2:1) and then azeotroped with toluene twice before using it in the next step.

9-Formyl-2-(Boc-amino)fluorene (3): In a 3 necked RBF, NaH (60% in oil; 330mmol, 3.3eq) was suspended in dry THF (50ml), a solution of 2-(Boc-amino)fluorene from step 2 (28g; 100mmol) in dry THF (230ml) was added dropwise over 20 minutes. A thick, yellow slurry was observed, and the mixture stirred for 10 minutes at RT under nitrogen. Ethyl formate (20.1ml, 250mmol, 2.5eq) was added dropwise (caution: gas evolution). The slurry turned to a pale brown solution. The solution was stirred for 20 minutes. The reaction was monitored by TLC (Rf=0.5, Hexane/Ethyl acetate 1:1) and when only traces of starting material was observed, it was quenched with iced water (300ml). The mixture was evaporated under reduced pressure until most of the THF has been removed. The resulting mixture was treated with acetic acid to pH=5. The white precipitate obtained was dissolved in ethyl acetate and the organic layer separated. The aqueous layer was extracted with ethyl acetate and all the organic layer combined and washed with saturated sodium bicarbonate, brine and dried over MgSO₄. After filtration and solvent removal, a yellow solid was obtained. This material was used in the next step.

9-Hydroxymethyl-2-(Boc-amino)fluorene (4): Compound 3 was suspended in MeOH (200ml) and sodium borohydride was added portion wise over 15 minutes. The mixture was stirred for 30 minutes (caution: exothermic reaction and gas evolution). The reaction was monitored by TLC (Rf=0.5, Hexane/EtOAc 1:1) and was completed. Water (500ml) was added and the pH adjusted to 5 with acetic acid. The work up involved extraction twice with ethyl acetate, washing the combined organic layers with sodium bicarbonate and brine, drying over MgSO₄, filtration and concentration to dryness. The crude obtained was purified by flask chromatography using Heptane/EtOAc (3:1) yielding a yellow foam (36g, 97.5% purity, traces of ethyl acetate and diethyl ether observed in the ¹H-NMR).

9-Hydroxymethyl-2-aminofluorene (5): compound 4 was added to an ice cold solution of 4N HCl in dioxane. The reaction mixture was allowed to reach RT and stirred overnight. A pale yellow precipitate was obtained. The suspension was cold at 0°C and stirred further for 5 hours. After this time, the solid was filtered and washed thoroughly with DCM (5x30ml). After drying, a pale yellow solid was obtained (20g, 96.5% purity) with an overall yield of 80% over 3 steps.

9-Hydroxymethyl-2-(amino-3-maleimidopropionate)fluorine (6): 9 Hydroxymethyl-2-aminofluorene (5, 5.5g, 26mmol) and maleimidopropionic anhydride (6.93g, 26mmol) were placed in a 250ml RBF equipped with a stirrer, a reflux condenser and a nitrogen bubbler. Reaction mixture was refluxed at 85°C for 25 hours. TLC (Rf=0.25, Hexane/EtOAc 1:4) showed reaction completion after this time. The reaction mixture was concentrated under vacuum to afford a yellow solid. The product was purified by column chromatography.

MAL-Fmoc-NHS (7): A clean dry 500ml RBF with overhead agitation was charged triphosgene (1.58g, 0.35eq.) in dry THF (55ml) to form a solution at ambient. The solution was cooled to about 0°C with an ice/water bath, and a solution of NHS (0.67g, 0.38eq) in dry THF (19ml) was added dropwise over 10 minutes under nitrogen at 0°C. The resultant solution was stirred for 30 minutes. A further portion of NHS (1.34g, 0.77eq) in dry THF (36ml) was added dropwise at 0°C over 10 minutes and stirred for 15 minutes.

Compound 6 (5.5g, 1eq), dry THF (55ml) and pyridine (3.07ml, 2.5eq) were stirred together to form a suspension. This was added to the NHS solution in portions at 0-5°C and then allowed to go to RT by removing the ice bath. After 20 hours, the reaction was stopped (starting material still present, if the reaction is pushed to completion a dimmer impurity has been observed). The reaction mixture was filtered and to the filtrate, 4% brine (200ml) and EtOAc (200ml) were added. After separation, the organic layer was washed with 5% citric acid (220ml) and water (220ml). The organic layer was then concentrated to give 7.67g of crude MAL-Fmoc-NHS. The material was purified by column chromatography using a gradient cyclohexane/EtOAc 70:30 to 40:60. The fractions containing product were concentrated under vacuum to give 3.47g (45%) of MAL-Fmoc-NHS.

MAL-FMS-NHS (test reaction): to a solution of MAL-Fmoc-NHS (100mg, 0.2mmol) in trifluoroacetic acid (10ml), chlorosulfonic acid (0.5ml) was added. After 15 minutes, ice-cold diethyl ether (90ml) was added and the product precipitated. The material was collected by centrifugation, washed with diethyl ether and dried under vacuum. 41.3mg (35%) of beige solid was obtained.

### Stage 3 - Conjugation

PEG-Fmoc-OXM conjugation: Conjugation with PEG, Fmoc and OXM were performed on a molar ratio of 1:1:1 e.g. PEG40-SH (44mg, in 4.4ml water equivalent to 1.0 µmol) added to peptide (4.5mg, equivalent to 1.0 µmol) and NaHCO3 (1M, 0.1ml) added. Fmoc (Almac, 10mg/ml in DMF, 50µl) added with stirring. Reaction stirred for 24h at RT.

PEG-FMS-OXM conjugation: All conjugations were performed at 1:1:1 molar ratio between the PEG the linker and OXM with the following reagents: PEG₄₀-SH and PEG₃₀-SH (NOF), FMS (Almac), EMCS (Termo Scientific), OXM (Almac). PEG₄₀-SH was dissolved in 0.1M sodium phosphate buffer (Sigma) pH 7.2 to a concentration of 10mg/mL. The solution was added to one equivalent of purified OXM peptide (Almac). MAL-FMS-NHS (Almac) linker was dissolved in DMF to a concentration of 10mg/mL one equivalent added to the reaction. The mixture was stirred for 30 minutes. The solution was neutralised to pH 4 using glacial acetic acid (Fisher). The neutralised mixture was filtered (0.45µm) and separated using preparative chromatography. The reaction mixture was filtered and purified by preparative HPLC (Phenomenex Luna C18) lyophilized and stored frozen.

The chromatographic parameters were as follows: Column: Phenomenex Luna C18(2) 250mm x 30mm, 15µm prep, 100A; Mobile Phase A: water (MilliQ) + 0.1% v/v TFA (Applied Biosystems); Mobile Phase B: water/acetonitrile (Rathburn) (25:75) + 0.1% v/v TFA (Applied Biosystems); UV Detection: 214nm; Gradient: 10%B to 65%B over 41 minutes; and Flow: 43mL/min.

OXM content was determined using amino acid analysis (AAA) or basic hydrolysis. A defined quantity of lyophilized OXM conjugate was dissolved in water at a concentration of 20 mg/ml. The absorbance at 280nm was than determined, and the concentration according to the absorbance at 280nm was calculated using ε280=29,700. The concentration of the peptide was accurately quantitated by acid-hydrolyzing an aliquot followed by quantitative amino acid analysis; the ideal fraction is the one having close agreement between the calculated absorbance at 280nm and the peptide content.

### Induction of cAMP cell based assay

CHO-K1 cells over-expressing GLP-1 receptor (Millipore HTS163C2) were seeded in 96 wells half-area white plate (Greiner) at a density of 200,000 cells/ml and incubated for 24 hours at 37°C. The cells were incubated with escalating concentrations of OXM (ALMAC), PEG40-EMCS-OXM and PEG40-Fmoc-OXM with or without rat serum 1% (Bio reclamation). Cells' cAMP concentrations were quantified by HTRF assay (Cisbio 62AM4PEB), and the EC50 parameter was analyzed by PRISM software.

### Pharmacokinetic study

The pharmacokinetic profile of PEG₄₀-Fmoc-OXM was assessed as follows: Male Wistar rats were administrated intravenously (IV) or subcutaneously (SC) with a single dose of native OXM (n=9, 278µg/kg) or with PEG₄₀-Fmoc-OXM (n=6, 278µg/kg peptide equivalent). Cohorts of 3 animals per group were bled at alternating time points. OXM serum concentration was analyzed using a commercial ELISA kit (Cat# S-1393, Bachem).

### IP glucose tolerance test

C57BL/6 male mice were fasted overnight and weighed, and blood glucose levels were measured by tail vein sampling using a handheld glucometer. Mice were IP injected with PBS (vehicle), OXM (333nmol/kg), PEG₄₀-EMCS-OXM (non-reversible pegylated OXM, 333nmol/kg body weight peptide content) and PEG40-Fmoc-OXM (202nmol/kg body weight peptide content) and PEG₄₀-Osu (546nmol/kg) as control. Glucose (1.5gr/kg) was administered IP either 15min after test article administration (vehicle, OXM and PEG₄₀-Osu) or 120 min after PEG₄₀-Fmoc-OXM administration. Blood glucose levels were measured by tail vein sampling prior to glucose administration and 10, 20, 30, 60 and 120 min after glucose administration using a handheld glucometer.

### Diet-induced obesity mice model

Study 1: C57BL/6J mice (4-6 weeks of age, Harlan UK Limited, Bicester, Oxon, UK), were group housed upon arrival in polypropylene cages. All animals had free access to a high fat diet (D12451; 45% of kcal derived from fat; Research Diets, New Jersey, USA) and tap water at all times. Animals were maintained on a normal phase 12 h light-dark cycle (lights on 07:00). Animals were exposed to the appropriate diet for at least 6 months (until the average body weight was approximately 50g). Subsequently, animals were singly housed in polypropylene cages for a further two-week period and placed on reverse phase lighting (lights off for 8 h from 09:30 - 17:30 h). During the second week of single housing, animals began a once-daily handling protocol and a 7-day baseline period. Subsequently, mice were dosed with vehicle or test drug as given below in Table 1:

**Table 1**

| **Group** | **Treatment (sc)** | **Frequency** | **n** |
|---|---|---|---|
| A | Vehicle (PBS) | b.i.d | 10 |
| B | OXM 5000nmol/kg body weight(PBS) | b.i.d | 10 |
| C | Sibutramine 20 mg/kg (PBS) | b.i.d | 10 |
| D | PEG40-FMS-OXM 5000nmol/kg body weight(citrate buffer) | Days 1, 3, 5, 7 | 10 |
| E | 556 mg/kg (27.8 mg/ml) PEG-SH (citrate buffer) | Days 1, 3, 5, 7 | 10 |

Measurements of body weight and food intake were performed daily until Day 8. The final measurement of body weight was carried out on Day 12. OXM and Sibutramine were formulated in PBS while PEG40-FMS-OXM and PEG-SH were formulated in 147mM NaCl 10mM citrate buffer pH 6. OXM content in PEG40-FMS-OXM was determined by basic hydrolysis.

Study 2: Study 2 was carried out as described for Study 1. Following a baseline period, animals were dosed according to the following design described in Table 2:

**Table 2**

| **Group** | **Treatment (SC)** | **Frequency** | **n** |
|---|---|---|---|
| A | Vehicle (PBS) | b.i.d | 8 |
| B | 3XM 5000nmol/kg body weight(PBS) | b.i.d | 8 |
| C | PEG40-FMS-OXM 1000nmol/kg body weight (citrate buffer) | Day 1,4,7 | 9 |
| D | PEG40-FMS-OXM 5000nmol/kg body weight (citrate buffer) | Day 1,4,7 | 9 |
| E | PEG40-FMS-OXM 8000nmol/kg body weight (citrate buffer) | Day 1,7 | 9 |
| F | PEG40-EMCS-OXM 1000nmol/kg body weight (citrate buffer) | Day 1,4,7 | 9 |
| G | PEG40-EMCS-OXM 5000nmol/kg body weight (citrate buffer) | Day 1,4,7 | 9 |
| H | PEG40-EMCS-OXM 8000nmol/kg body weight (citrate buffer) | Day 1,7 | 9 |
| I | PEG30-FMS-OXM 5000nmol/kg body weight (citrate buffer) | Day 1,4,7 | 9 |
| J | PEG40-SH (citrate buffer) | Day 1,4,7 | 9 |
| K | Sibutramine | b.i.d | 8 |

Measurements of body weight and food intake were performed daily until Day 14.

Study 3: Study 3 was carried out as described for Study 1&2 with one difference, the mice at the beginning of the experiment were weight 45-46g. Following a baseline period, animals were dosed according to the following design described in Table 3:

**Table 3**

| **Group** | **Treatment (sc)** | **n** |
|---|---|---|
| A | PEG5-FMS-OXM 6000 nmol/kg: Day 1, 8,15 | 7 |
| B | PEG30-FMS-6000 nmol/kg: Day 1, 8, 15 | 7 |
| C | PEG40-FMS-OXM 6000 nmol/kg: Day 1, 8, 15 | 7 |
| D | PEG60-FMS-OXM 6000 nmol/kg: Day 1, 8,15 | 7 |
| E | Vehicle (PBS sc) | 7 |
| F | Liraglutide (200 µg/kg bid) in PBS | 7 |

### Data and statistical analysis

OXM and Sibutramine were formulated in PBS while PEG40-EMCS-OXM, PEG40-FMS-OXM and PEG-SH were formulated in 147mM NaCl 10mM citrate buffer pH 6. OXM content in PEG40-FMS-OXM and PEG40-EMCS-OXM were determined by AAA.

Body weight and food intake are expressed as mean values ± SEM. Body weight, body weight gain, daily and average food intake data and cumulative food intake were analysed by ANCOVA with baseline as a covariate, followed by appropriate comparisons (two-tailed) to determine significant differences from the control group. P<0.05 is considered to be statistically significant. Baseline was Day 1 value for body weight or the average food or water consumption over the baseline period.

### EXAMPLE 1

### Synthesis and characterization of PEG-Fmoc-OXM

OXM peptide was synthesized by the solid phase method employing the Fmoc-strategy throughout the peptide chain assembly. The peptide was purified by preparative HPLC using Phenomenex Luna C18 (250 x 30mm) column by applying gradient between solution A (0.1% TFA+H₂O) and B (0.1% TFA + MeCN). Peptide purity was above 95%, the molecular weight was 4449 Da (measured by MALDI). Conjugation of OXM peptide to PEG₄₀-SH through Fmoc linker was performed in the presence of NaHCO₃. The reaction mixture was stirred for 24h at RT followed by filtration and purification by preparative HPLC (Jupiter C5). Conjugate molecular weight was analyzed by MALDI and OXM peptide content was analyzed by AAA. The average OXM peptide content was 189µg OXM per 1mg PEG₁₀-Fmoc-OXM conjugate 132.4µg OXM per 1mg PEG₂₀-Fmoc-OXM conjugate, 61.7µg OXM per 1mg PEG₄₀-Fmoc-OXM conjugate and 40µg OXM per 1mg PEG₄₀-FMS-OXM conjugate.

### EXAMPLE 2

### Pharmacokinetic profile of PEG10-Fmoc-OXM, PEG20-Fmoc-OXM and PEG40-Fmoc-OXM compared to native OXM

The pharmacokinetic profile of OXM compared to PEG₁₀-Fmoc-OXM and PEG₂₀-Fmoc-OXM was evaluated in male Wistar rats. Animals were administrated with a single SC injection of native OXM (278µg/kg peptide), PEG₁₀-Fmoc-OXM (278µg/kg peptide content) or PEG₂₀-Fmoc-OXM (278µg/kg peptide content). The serum concentration of the compound at indicated time intervals was measured (commercial ELISA, PK profile shown in Figure 1 and conventional noncompartmental PK parameters are summarized in Table 3). Reversible pegylation of OXM conjugated to both PEG₁₀ and PEG₂₀ resulted in prolongation of the half-life of native OXM (0.15hr for native OXM; 16.16hr for PEG₁₀-Fmoc-OXM and 27.38hr for PEG₂₀-Fmoc-OXM). Exposure, as reflected by the AUC parameter, was increased by about ~450-fold for PEG₁₀-Fmoc-OXM and about -2210 for PEG₂₀-Fmoc-OXM. Thus, reversible conjugation of OXM to PEG₂₀ resulted in a more prolonged effect compared to PEG₁₀. In order to further characterize the PK profile of OXM reversibly conjugated to PEG₄₀ through Fmoc linker, male Wistar rats were injected IV or SC with native OXM or PEG₄₀-Fmoc-OXM (278µg/kg peptide content) and serum concentration at indicated time points were analyzed (using commercial ELISA, PK profile shown in Figure 2 and conventional noncompartmental PK parameters are summarized in Table 4). The results indicated that reversible pegylation prolong the half life of OXM peptide by 100 fold, and increase the exposure significantly as reflected by AUC parameter, Moreover, the bioavailability of the native peptide was only 4.37% while administration of PEG₄₀-Fmoc-OXM resulted in 84% bioavailability.

**Table 3: Non-compartmental PK parameters of OXM and PEG₁₀-Fmoc-OXM and PEG₂₀-Fmoc-OXM following SC administration in rats.**

| | **AUC** hr*ng/ml | **T1/2 term.** hr | **MRT** hr |
|---|---|---|---|
| OXM | 3.2 | 0.15 | 0.3 |
| PEG₁₀-Fmoc-OXM | 1456 | 16.16 | 20.6 |
| PEG₂₀-Fmoc-OXM | 7079 | 27.38 | 37.2 |

**Table 4: PK parameters of OXM and PEG₄₀-Fmoc-OXM following IV or SC administration in rats.**

| | **Route of Administration** | **AUC** hr*ng/ml | **T1/2 term.** hr | **MRT** hr | **F** % |
|---|---|---|---|---|---|
| **OXM** | IV | 72.44 | 0.44 | 0.414 | 100 |
| | SC | 3.34 | 0.69 | 0.913 | .374 |
| **PEG₄₀-Fmoc-OXM** | IV | 435,73 | 23.3 | 24.3 | 100 |
| | SC | 656,65 | 30.4 | 57.7 | 4.68 |

### EXAMPLE 3

### Induction of cAMP by OXM and reversible pegylated OXM

In order to assess the in vitro activity of the OXM compared to PEG40-Fmoc-OXM, and PEG40-EMCS-OXM (non -reversible pegylated OXM), CHO-K1 cells over-expressing GLP-1 receptor were incubated with escalating concentrations of the different compound followed by cAMP quantitation. Native OXM demonstrated improved activity compared to PEG₄₀-Fmoc-OXM and PEG₄₀-EMCS-OXM which had comparable in-vitro activity (EC50 of 2.53x10-9, 2.07x10-6 and 5.87x10-7 for OXM, PEG40-EMCS-OXM and PEG40-Fmoc-OXM respectively, Figure 3). Importantly, OXM pegylation didn't abrogate completely the GLP-1 receptor activation induced by OXM. In addition, while incubation of OXM in serum resulted in reduced activity, probably due partial proteolysis of the peptide, comparable activities in the present and absence of rat serum were obtained for PEG₄₀-Fmoc-OXM and PEG₄₀-EMCS-OXM, suggesting that pegylation masks potential proteolysis sites on OXM.

### EXAMPLE 4

### Reversible pegylated long acting OXM induced glucose tolerance

In order to evaluate the in vivo activity of the OXM or PEG₄₀-Fmoc-OXM, the IPGTT model was applied. Overnight fasted C57BL/6 mice were injected IP with OXM peptide or PEG₄₀-Fmoc-OXM followed by IP injection of glucose and measurement of blood glucose levels from the tail vein by glucometer. OXM (333nmol/kg), PEG₄₀-EMCS-OXM (non - reversible pegylated OXM, 333nmol/kg body weight peptide content) and PEG40-Fmoc-OXM (202nmol/kg body weight peptide content) were administrated IP 15 min (OXM and PEG₄₀-EMCS-OXM) or 2 hrs PEG₄₀-Fmoc-OXM, prior to glucose IP injection (1.5gr/kg). The induction of glucose tolerance was compared to vehicle group. As control to the effect of PEG₄₀, a control group was administrated with PEG₄₀-Osu (546nmol/kg). While OXM peptide had a minor effect on the glucose tolerance compared to vehicle group, administration of PEG₄₀-Fmoc-OXM having even lower OXM molar content resulted in induced glucose tolerance (Figure 4). Surprisingly, administration of non-reversible pegylated resulted in induction of glucose tolerance suggesting that pegylated OXM is pharmacologically active in-vivo.

### EXAMPLE 5

### Reversible pegylated long acting OXM reduce body weight and inhibit food intake in DIO mice

The pharmacological activity of OXM was further evaluated in DIO mice following SC injection of native OXM, and reversibly-pegylated OXM. In study 1, male DIO mice (n=10 per group) were administered with either 5000nmol/kg body weight of OXM b.i.d or PEG₄₀-FMS-OXM containing 5000nmol/kg body weight OXM every other day for seven days of dosing. Body weight and food intake were measured daily for 8 days with a final measurement of body weight on day 12. Twice a day injection of OXM resulted in a moderate reduction in both body weight (6% weight loss on Day 8 compared to vehicle control group) and statistically significant inhibition of food intake. On the other hand, administration of PEG₄₀-FMS-OXM having the same OXM peptide content per dose but injected every other day resulted in a marked weight loss (24% weight loss on Day 8 compared to PEG-SH control group) and manifested a substantial inhibition of food intake (Figure 4). Sibutramine, neurotransmitter reuptake inhibitor, which was used as positive control reduced body weight by 15.6%. Of note, the reduction of body weight in the PEG₄₀-FMS-OXM group was consistent until the last measurement on Day 12 which is 5 days following the last dose, indicating a long lasting behavior of reversibly-pegylated OXM (Figure 5).

Since PEG₄₀-EMCS-OXM induced glucose tolerance in the IPGTT model it was important to compare the efficacy of non-reversible pegylated OXM to reversibly-pegylated OXM in the context of body weight and food intake. Consequently, a follow up study was designed to address this issue (study 2 in materials and methods). While administration of 5000nmol/kg body weight of PEG₄₀-FMS-OXM every 3 days (total of 3 injections) resulted in substantial reduction of body weight, injection of 5000nmol/kg body weight PEG₄₀-EMCS-OXM in the same frequency resulted in a negligible effect on body weight. Remarkably, single injection on Day 1 of 8000nmol/kg body weight of PEG₄₀-FMS-OXM resulted in apparent weight reduction for 6 days. Surprisingly, administration of 5000nmol/kg body weight of PEG₃₀-FMS-OXM resulted in elevated reduction in body weight indicating an improved efficacy compared to PEG₄₀-FMS-OXM (Figure 6).

OXM is a potential peptide for the treatment of metabolic disorders such as diabetes and obesity, as demonstrated by the weight lost obtained by native OXM in over weight and obese healthy subject (Wynne et al, 2005). Yet, due to the short half-life of the peptide and its low stability in-vivo, repeated daily administrations of supra-physiological doses are required in order to achieve a pharmacological effect in humans. This patent provides effective means for stabilizing OXM in physiological conditions by reversibly pegylating the acting OXM thus rendering long-acting. Unexpectedly, the modified OXM-the pegylated version is active and is not a mere pro-drug.

Reversibly-pegylated OXM demonstrated superior pharmacokinetic profile in rats with a substantial increase in the exposure and elongated half-life compared to native OXM. When comparing the effect of PEGs with various molecular weights reversibly conjugated to OXM on OXM-PK profile, PEG₄₀ -conjugate demonstrated a superior prolonging effect compared to PEG₁₀ or PEG₂₀. Therefore, the PEG₄₀ was further evaluated in pharmacological studies (Figures 1 and 2). Importantly, the bioavailability of OXM was significantly increased from 4.37% to 84.6% following SC administration of PEG40-Fmoc-OXM, contributing to the increased exposure of reversibly-pegylated peptide (Table 2). PEG₄₀-Fmoc-OXM improved glucose tolerance as compared to native OXM as assessed in overnight fasted C57BL/6 mice IPGTT model. In this model a non-reversible pegylated OXM conjugated to PEG₄₀ (PEG₄₀-EMCS-OXM) demonstrated comparable glucose tolerance induction activity to the PEG40-Fmoc-OXM. This result further supported by the in-vitro activity observed for PEG₄₀-EMCS-OXM and PEG₄₀-Fmoc-OXM in which conventional pegylation of OXM does not completely abolish the binding of OXM to its receptor, a phenomenon observed for pegylated peptides due to steric interference, and consequently does not result in overall loss of biological activity (Figures 3 and 4).

Next, the effect of PEG₄₀-FMS-OXM on body weight and food intake was evaluated in DIO mice compared to native OXM. SC injection of 5000nmol/kg of native OXM administered twice daily resulted in a moderate reduction in body weight and food intake following 7 days of dosing. In contrast, injection of 5000nmol/kg PEG₄₀-FMS-OXM every other day resulted in a marked reduction in both body weight and food intake (6% and 24.9% reduction in body weight for OXM and PEG₄₀-FMS-OXM respectively, Figure 5) compared to control on Day 8. In conclusion, PEG₄₀-FMS-OXM exhibited a prolonged anti-obesity effect and improved efficacy considering that the cumulative dose of OXM administered during the study for PEG₄₀-FMS-OXM was almost 4 times lower compared to the group administered with native OXM.

Non-reversible pegylated OXM, PEG₄₀-EMCS-OXM, was shown to improve glucose tolerance in IPGTT test. It was therefore imperative to evaluate the food regulation activity of conventional pegylation compared to reversibly pegylated OXM and native OXM in the DIO model. Administration of 5000nmol/kg of PEG₄₀-EMCS-OXM every three days resulted in a negligible reduction in body weight although inhibition of food intake was evident up to 3 days post dosing (Figure 6). The moderate inhibition of food intake probably results from the direct activity of OXM in the gastrointestinal tract and correlates with the peripheral activity observed in the IPGTT model. As OXM food regulation activity involves the crossing of the blood brain barrier and binding to receptors on neurons in the ARC, it is imperative that the ability of OXM to penetrate to the CNS will not be abolished. The observed peripheral bioactivity of PEG₄₀-EMCS-OXM as opposed to the lack of ability of this compound to reduce DIO mice body weight suggests that the covalent bond to the PEG moiety restrict the ability of PEG₄₀-EMCS-OXM to pass-through the BBB into the ARC which is the potential action site of OXM in the hypothalamus. In contrast, injection of 5000nmol/kg PEG₄₀-FMS-OXM in the same frequency markedly reduced body weight and inhibited food intake by 20% as measured on day 12. Remarkably, injection of 8000nmol/kg PEG₄₀-FMS-OXM once a week resulted in similar body weight reduction by 20%, indicating that in humans, significant weight lose can be achieved by once a week injection or even less frequent dosing of reversibly pegylated OXM.

The reversible pegylation strategy is aiming to overcome the loss of activity often observed in conventional pegylation while retaining the prolonging effect of the drug. In cases where the pegylated prodrug bioactivity is dramatically lost or even abolished the advantage in applying reversible pegylation was previously proven (United States Patent No. 7585837). Yet, it is unknown what will be the efficacy of a reversibly pegylated prodrug compared to covalently non-reversible pegylated drug that retain its biological activity. This is especially relevant as the PK profile of covalently pegylated peptide is expected to be superior compared to reversible pegylated peptide when assessing the conjugate blood concentrations, due to the slow release of the peptide from the conjugate. PEG₄₀-EMCS-OXM was shown to be active both in-vitro and in the IPGTT model in-vivo. Therefore, it was possible that this molecule will also induce satiety and reduce body weight following SC administration to mice. However, this was shown to be incorrect as PEG₄₀-EMCS-OXM fail to reduce body weight in DIO mice while PEG₄₀-FMS-OXM had a marked effect. Previous publication presented conflicting data re the contribution of the peripheral activity of OXM and the CNS activity of OXM. On the one hand, the anorectic actions of ip OXM were blocked by prior intra-ARC administration of the GLP-1R, exendin₉₋₃₉ indicating the importance of the CNS-related activity of OXM (Dakin et al 2004). Yet, an oral delivery of Bifidobacterium expressing OXM to overweight mice resulted in reduction in body weight while OXM was not detected in the plasma of this mice, suggesting that the direct activation of gastrointestinal cells is important for the weight loss activity of OXM (Long et al, 2010). As mentioned above the lack of information on mode of action of OXM and the impact of pegylation, it was impossible to predict what will be the efficacy of reversibly pegylated OXM compared to native OXM and covalently bound pegylated OXM.

The superior efficacy of PEG₃₀-FMS-OXM compared to PEG₄₀-FMS-OXM as shown in study 2 was surprising. Although the PK profile of PEG₃₀-FMS-OXM was not evaluated, PEG₄₀-FMS-OXM PK profile was clearly superior to PEG₁₀-FMS-OXM and PEG₂₀-FMS-OXM. It is possible that the use of PEG₃₀ present the favorable PEG size that on the one hand reduce significantly the renal clearance of the conjugated PEG₃₀-FMS-OXM, while facilitate OXM rate of hydrolysis from the conjugate that enable a sustained presence of OXM required for eliciting its pharmacological activities.

### Study 3

In this study the conjugation of OXM to PEG of various sizes was evaluated. As was shown in the previous studies, administration of PEG30-FMS-OXM and PEG40-FMS-OXM once weekly had a marked effect on body weight. Surprisingly, PEG5-FMS-OXM completely lost the ability to induce weight loss as compared to the vehicle group, while PEG60-FMS-OXM induced an even more pronounced reduction than PEG30-FMS-OXM. The difference in weight loss between PEG30-FMS-OXM and PEG40-FMS-OXM in this study was in the experiment variability range and it was not significant.

### EXAMPLE 6

### Improved Glycemic and Lipidemic Profiles in Obese Mice Treated with Reversible PEGylated OXM

### Materials and Methods

### Experimental Procedures for Diet Induced Obesity (DIO) Mouse Model:

The DIO model was carried out at RenaSci Ltd Company (Nottingham, UK). C57BL/6J mice (4-6 weeks of age, Harlan UK Limited, Bicester, Oxon, UK), were exposed to a high fat diet (D12451; 45% of kcal derived from fat; Research Diets, New Jersey, USA) for at least 6 months (until the average body weight is approximately 50g). Two weeks prior to drug administration, animals were singly housed and placed on reverse phase lighting (lights off for 8 h from 09:30 - 17:30 h). During the first week of single housing (handling period), animals began a once-daily handling protocol and during the second week (baseline period), they were dosed with the appropriate vehicle b.i.d. or once a week as they were dosed during the treatment period) by a subcutaneous route. 7 groups (n=8) of DIO mice were dosed for 29 days as follows:

| **Group** | **Treatment (SC)** | **Frequency** |
|---|---|---|
| A | PEG40-SH (662 mg/kg) | Once a week (1, 8, 15, 22, 29) |
| B | PEG40-EMCS-OXM (6,000nmol/kg) | Once a week (1, 8, 15, 22, 29) |
| C | PEG30-EMCS-OXM (6,000nmol/kg) | Once a week (1, 8, 15, 22, 29) |
| D | PEG40-FMS-OXM (6,000nmol/kg) | Once a week (1, 8, 15, 22, 29) |
| E | PEG30-FMS-OXM (6,000nmol/kg) | Once a week (1, 8, 15, 22, 29) |
| F | Vehicle (PBS) | b.i.d |
| G | OXM (6,000nmol/kg; PBS) | b.i.d |

During the baseline and the treatment period food intake, water intake and body weight were recorded daily. On days 1 and 22 after a two-week baseline, all the mice were overnight fasted. On days 2 and 23, the mice underwent an oral glucose tolerance test (OGTT). Each animal were dosed with vehicle or test compound and 60 minutes later were dosed with D-glucose (2 g/kg po). Baseline blood samples were taken immediately prior to dosing with vehicle or test compound (B1) and immediately before the glucose load (B2). Further blood samples were taken 10, 20, 30, 45, 60 and 120 minutes post glucose administration. All blood samples (approximately 20µl) were taken from the tail vein. Plasma samples were prepared and assayed for glucose (n = 2) and insulin (n = 1) using the Thermoelectron Infinity glucose reagent (TR15421) and Alpco mouse ultrasensitive insulin ELISA (80-INSMSU-E10), respectively. On Day 30, terminal plasma samples were collected (24 hours after the final dose on Day 29) by cardiac puncture and assayed for insulin, glucose, cholesterol and triglycerides using the mouse ultrasensitive insulin ELISA (80-INSMSU-E10), Thermoelectron Infinity glucose reagent (TR15421), Thermoelectron Infinity cholesterol reagent (TR13421) and the Sigma Triglyceride kit (TR0100). Final carcass weights were recorded after terminal blood sampling and carcasses frozen at -20°C.

### Experimental procedures for body composition studies:

Body fat, protein, water and ash levels of the carcasses were determined using standard chemical analysis techniques. Only fat, protein, water and ash content were measured, since other components (mainly carbohydrate) form less than 2% of total body composition. Carcass water was determined by freeze-drying the mouse carcasses to constant weight. Dried carcasses were then ground in a laboratory grinder ready for subsequent analyses. Carcass fat was determined on the freeze-dried samples using a modified Soxhlet extraction protocol (petroleum ether at 40-60°C) with a Tecator Soxtec 2050 system (Foss UK Ltd, Wheldrake, UK) according to the manufacturer's recommended protocol. Carcass protein was determined using a micro-Kjeldahl procedure on the freeze-dried samples using a Tecator 2012 digestion block and 2200 distilling unit (Foss UK Ltd). Residual carcass ash was determined by firing the freeze-dried samples at high temperatures using a muffle ashing furnace.

### Data and statistical analysis:

Body weights, food intake and water intake expressed as mean values ± SEM. Body weight, body weight gain, daily and average food and water intake data and cumulative food intake were analysed by ANCOVA with baseline as a covariate, followed by appropriate comparisons (two-tailed) to determine significant differences from the control group. P<0.05 is considered to be statistically significant. Baseline was Day 1 value for body weight or the average food or water consumption over the baseline period.

Terminal plasma insulin, cholesterol and triglycerides were analysed by general linear model with treatment as a factor and bleeding order and baseline body weight as covariates followed by appropriate comparisons (two-tailed) to determine significant differences from the relevant vehicle group. A log transformation and/or robust regression techniques were used if appropriate.

Data for each body composition parameter (fat, protein, water and ash) were presented as g/carcass and % total. Final carcass weights were also analysed as a direct comparison. The analysis was done by robust regression with treatment as a factor and body weight at baseline as a covariate, followed by appropriate multiple comparisons tests (two-tailed) to compare the effects of each treatment group with the relevant vehicle group.

### Results

A weekly injection of reversible PEG30 (PEG30-FMS-OXM (6,000 nmol/kg; citrate buffer)) or reversible PEG40 (PEG40-FMS-OXM (6,000 nmol/kg; citrate buffer)), during a 30-day period, provided 28% and 23% weight loss, respectively, compared to 17% weight loss for the group injected twice per day with native oxyntomodulin (Figure 7) - while the cumulative dosing of net oxyntomodulin injected with reversible PEG30 was only 8.6% for the 30-day period. Non-reversibly PEGylated OXM (PEG40-EMCS-OXM and PEG30-EMCS-OXM) were even less effective in reducing body weight.

Glucose tolerance in DIO mice after weekly injections with reversible PEGylated OXM (PEG30-FMS-OXM or PEG40-FMS-OXM) was comparable to the glucose tolerance elicited by a twice per day injection of native oxyntomodulin at Day 2 (Figure 8A) and at Day 23 (Figure 8B).

In addition, a once weekly administration of reversible PEGylated OXM improved the glycemic and lipidic profiles in DIO mice, demonstrated by a reduction in terminal glucose (Figure 9A), a reduction in terminal insulin (Figure 9B), a reduction in terminal cholesterol (Figure 9C), and a reduction in terminal glycerol (Figure 9D).

Finally, a body composition analysis of the DIO mice demonstrated that the weight loss demonstrated by mice treated with reversible PEGylated OXM resulted from a specific reduction in fat (Figure 10).

Taken together, reverse PEGylation was shown to be safe and tolerable in different toxicological rodent animal models. Reverse PEGylation also enables elongation of OXM half-life, while maintaining its potential to penetrate target tissues (e.g. penetrate the BBB).

Reversibly PEGylated OXM demonstrated superior long acting properties, supporting once weekly injection in humans. Reversibly PEGylated OXM reduced the body weight by a specific reduction in fat (Body Composition assessment). Reversibly PEGylated OXM improved the glycemic and lipidemic profiles. Reversibly PEGylated OXM is expected to provide long-term therapy for obesity and Type II Diabetes patients via its impressive effects on glycemic activity and fat loss.

### EXAMPLE 7

### Effect of reversible pegylated OXM on glucose level and insulin secretion

### Experimental procedures for Diet induced Obesity (DIO) mice model:

The DIO model was carried out at RenaSci Ltd Company (Nottingham, UK). 57BL/6J mice (4-6 weeks of age, Harlan UK Limited, Bicester, Oxon, UK), were exposed to a high fat diet (D12451; 45% of kcal derived from fat; Research Diets, New Jersey, USA) for at least 6 months (until the average body weight was approximately 50g). Two weeks prior to drug administration, animals were singly housed, where they began an acclimation period. On the first week, the handling period, animals began a once-daily handling protocol and during the second week, the baseline period, they were dosed with the appropriate vehicle; (b.i.d or once a week as they were dose during the treatment period) by a subcutaneous route. During the baseline and the treatment period food intake, water intake and body weight were recorded daily. On the morning of day 1 the mice were dosed followed by an overnight fasting. On days 2, 24h following administration (groups A-E) or prior to the morning dosing (groups F-H), the mice were sampled for fasting glucose and fasting insulin. All blood samples (approximately 20µl) were taken from the tail vein. Plasma samples were prepared and assayed for glucose (n = 2) and insulin (n = 1) using the Thermoelectron Infinity glucose reagent (TR15421) and Alpco mouse ultrasensitive insulin ELISA (80-INSMSU-E10), respectively.

### Results

In this set of experiments two independent *in vivo* studies were carried out. The first experiment included 8 groups (n=8) of DIO mice that were dosed for 2 days as follows:

| **Group** | **Treatment (SC)** | **Frequency** |
|---|---|---|
| A | PEG40-SH (662 mg/kg) | Single injection on day 1 |
| B | PEG40-EMCS-OXM (6,000nmol/kg) | Single injection on day 1 |
| C | PEG30-EMCS-OXM (6,000nmol/kg) | Single injection on day 1 |
| D | PEG40-FMS-OXM (6,000nmol/kg) | Single injection on day 1 |
| E | PEG30-FMS-OXM (6,000nmol/kg) | Single injection on day 1 |
| F | Vehicle (PBS) | b.i.d |
| G | OXM (6,000nmol/kg; PBS) | b.i.d |
| H | Liraglutide (200µg/kg) | b.i.d |

The second experiment included 7 groups (n=7) of DIO mice that were dosed for 2 days as follows:

| **Group** | **Treatment (SC)** | **Frequency** |
|---|---|---|
| A | PEG60-SH (947.4 mg/kg) | Single injection on day 1 |
| B | PEG5-FMS-OXM 6000 nmol/kg | Single injection on day 1 |
| C | PEG30-FMS-6000 nmol/kg | Single injection on day 1 |
| D | PEG40-FMS-OXM 6000 nmol/kg | Single injection on day 1 |
| E | PEG60-FMS-OXM 6000 nmol/kg | Single injection on day 1 |
| F | Vehicle (PBS) | b.i.d |
| G | Liraglutide (200 µg/kg bid) in PBS | b.i.d |

In both studies administration of a single dose of all PEG-OXM variants: PEG40-EMCS-OXM, PEG30-EMCS-OXM, PEG40-FMS-OXM, PEG30-FMS-OXM (Exp. #1) or PEG30-FMS-OXM, PEG40-FMS-OXM and PEG60-FMS-OXM (Exp. #2) produced marked and significant reductions in fasting glucose when compared to vehicle (figures 11 and 12). In experiment #1 the vehicle group (PEG40-SH) exhibits glucose level of 9.5 mM while the PEG-OXM treated groups exhibit glucose level of 5.18 to 5.8 mM. The same reduction of glucose level was obtained also for PEG-OXM treated group in experiment #2 (except PEG5-OXM group) that showed reduction of glucose from 11.9 mM of vehicle group to 5-5.7mM of PEG-OXM treated groups. This effect was associated with reduction in fasted plasma insulin levels in experiment #1 from 2.8 ng/ml of vehicle group to 1.4-1.9 ng/ml of PEG-OXM treated groups as shown in Figure 11. In Experiment #2 fasted insulin level was 0.99 ng/ml while fasted insulin level of PEG-OXM (except PEG5-OXM) was 0.78 to 0.91 ng/ml.

Liraglutide in both of the experiments significantly reduced fasting glucose when compared to vehicle (PBS); 9.3 mM of vehicle was decreased to 6.06 mM in experiment.#1 and 11.5 mM of vehicle was decreased to 6.7 mM in experiment. #2. Together with this reduction of glucose this treated group exhibited significant increase in plasma insulin from 2.5 ng/ml of vehicle to 4.4 ng/ml in experiment. #1 and from 1.98 ng/ml to 3 ng/ml in experiment. #2. OXM native peptide was analyzed in experiment. #1 and did not show any significant difference in glucose and insulin levels as compared to the vehicle, probably due to its short serum half-life and very rapid clearance from the body.

The results from these two independent experiments in DIO mice reveal that PEG-OXM compounds induce significant reduction of glucose level but without increasing insulin level as was observed following Liraglutide administration, and as expected from previously data that had been shown for OXM native peptide. This unexpected reduction of glucose level together with reduction of fasted insulin levels indicates that a single dose of PEG-OXM lead to increasing the sensitivity of the animals to insulin already following acute exposure and not due to chronic treatment.

## Claims

1. A compound of the formula (X)n-Y, wherein Y is dual GLP-1/Glucagon receptor agonist consisting of an oxyntomodulin (OXM) peptide having the amino acid sequence of SEQ ID NO: 1 and bearing a free amino or hydroxyl group, and wherein X is a radical of formula (i) 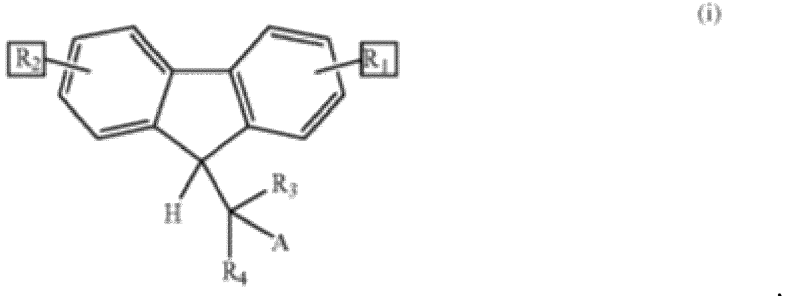 wherein
R₁ is a polyethylene glycol (PEG) moiety;
R₂ is either hydrogen or is -SO₃H at position 2 of the fluorene ring;
R₃ and R₄ are each hydrogen;
A is -OCO-, wherein the radical of formula (i) is linked to an amino or hydroxyl group of the drug Y; and
n is an integer of at least one.

2. The compound of claim 1, wherein the PEG moiety is a linear PEG.

3. The compound of claim 1, wherein the PEG moiety is a branched PEG.

4. The compound of claim 1, wherein the PEG moiety is a 30 kDa, 40 kDa or 60 kDa PEG.

5. A compound of claim 1, wherein said compound is prepared from a MAL-Fmoc-NHS having the formula: 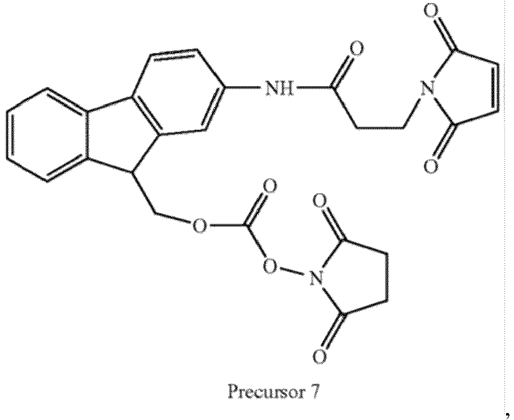 wherein said compound is designated as (PEG-Fmoc-)n-OXM.

6. A compound of claim 1, wherein said compound is prepared from a MAL-FMS-NHS having the formula: 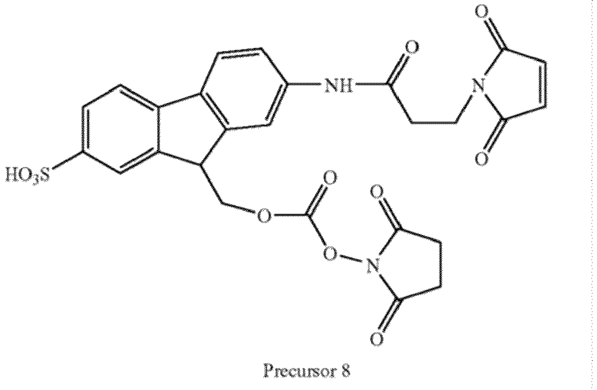 , wherein said compound is designated as (PEG-FMS-)n-OXM.

7. The compound according to claim 5, wherein Y is linked to the Fmoc radical through an amino group.

8. The compound according to claim 6, wherein Y is linked to the FMS radical through an amino group.

9. The compound according to claims 7 or 8, wherein said amino group is the amino terminus of said oxyntomodulin.

10. The compound according to claims 7 or 8, wherein the PEG is linked to the FMS or Fmoc radical through a sulfhydryl group.

11. A pharmaceutical composition comprising a compound according to any of claims 1-10, and a pharmaceutical acceptable carrier.

12. A method for the preparation of oxyntomodulin having an extended biological half-life, consisting of the step of conjugating oxyntomodulin, a polyethylene glycol polymer (PEG polymer) and 9-fluorenylmethoxycarbonyl (Fmoc) or 2-sulfo-9-fluorenylmethoxycarbonyl (FMS) in a molar ratio of 1:1:0.5 to 1:1:3.5, to form a compound of claim 1.

13. The compound according to any of claims 1-10, or the pharmaceutical composition of claim 11, for use in inducing glucose tolerance and/or improving glycemic control, or for use in reducing food intake and/or reducing body weight, in a subject in need thereof.

14. The compound of claim 1 for use in treating diabetes mellitus, diabetes mellitus associated with obesity, obesity, an eating disorder, or a metabolic disorder, in a subject.

15. The compound according to any of claims 1-10, or the pharmaceutical composition of claim 11, for use as a medicament.

16. The compound of claim 1, wherein R₂ is -SO₃H at position 2 of the fluorene ring, and the PEG moiety is a 40 kDa PEG.

## Patentansprüche

1. Verbindung der Formel (X)n-Y, wobei Y ein dualer GLP-1-/Glucagonrezeptoragonist ist, der aus einem Oxyntomodulinpeptid (OXM-Peptid) mit der Aminosäuresequenz SEQ ID NO: 1 und das eine freie Amino- oder Hydroxylgruppe trägt besteht, wobei X ein Radikal der Formel (i) ist 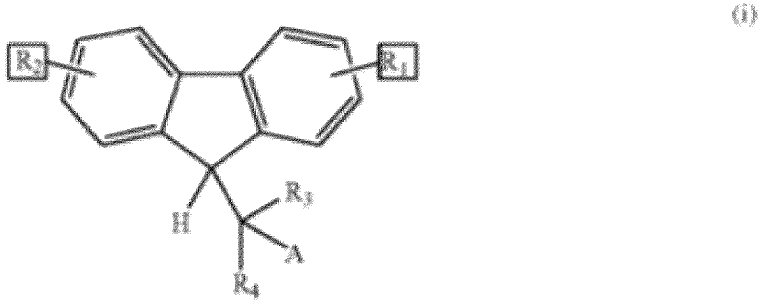 wobei
R₁ ein Polyethylenglycolrest (PEG-Rest) ist;
R₂ entweder Wasserstoff oder -SO₃H an Position 2 des Fluorenrings ist;
R₃ und R₄ jeweils Wasserstoff ist;
A -OCO- ist, wobei der Radikal der Formel (i) mit einer Amino- der Hydroxylgruppe des Arzneimittels Y verlinkt ist; und wobei
n eine ganze Zahl von mindestens 1 ist.

2. Verbindung nach Anspruch 1, wobei der PEG-Rest ein lineares PEG ist.

3. Verbindung nach Anspruch 1, wobei der PEG-Rest ein verzweigtes PEG ist.

4. Verbindung nach Anspruch 1, wobei der PEG-Rest ein PEG von 30 kDa, 40 kDa oder 60 kDa ist.

5. Verbindung nach Anspruch 1, wobei die Verbindung aus einem MAL-Fmoc-NHS hergestellt wird, welches die folgende Formel hat: 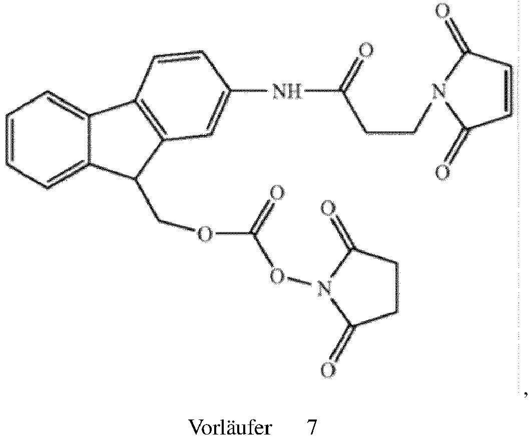 wobei die Verbindung als (PEG-Fmoc-)n-OXM bezeichnet wird.

6. Verbindung nach Anspruch 1, wobei die Verbindung aus einem MAL-FMS-NHS hergestellt wird, welches die folgende Formel hat: 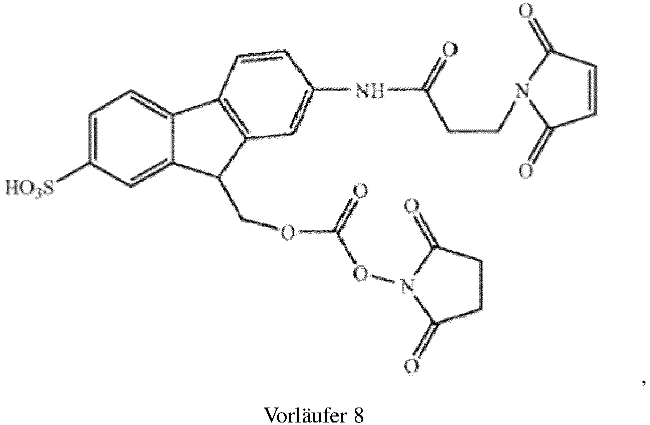 wobei die Verbindung als (PEG-FMS-)n-OXM bezeichnet wird.

7. Verbindung nach Anspruch 5, wobei Y durch eine Aminogruppe mit dem Fmoc-Radikal verlinkt ist.

8. Verbindung nach Anspruch 6, wobei Y durch eine Aminogruppe mit dem FMS-Radikal verlinkt ist.

9. Verbindung nach Anspruch 7 oder Anspruch 8, wobei die Aminogruppe der Aminoterminus von Oxyntomodulin ist.

10. Verbindung nach Anspruch 7 oder Anspruch 8, wobei das PEG durch eine Sulfhydrylgruppe mit dem FMS oder Fmoc-Radikal verlinkt ist.

11. Pharmazeutische Zusammensetzung, umfassend eine Verbindung nach einem der Ansprüche 1-10 und einen pharmazeutisch annehmbaren Träger.

12. Verfahren zur Herstellung von Oxyntomodulin, welches eine verlängerte biologische Halbwertszeit hat, das aus dem Schritt Konjugieren von Oxyntomodulin, einem Polyethylenglycolpolymer (PEG-Polymer) und von 9-Fluorenylmethoxycarbonyl (Fmoc) oder 2-Sulfo-9- fluorenylmethoxycarbonyl (FMS) in einem molaren Verhältnis von 1:1:0,5 bis 1:1:3,5 besteht, um eine Verbindung nach Anspruch 1 zu bilden.

13. Verbindung nach einem der Ansprüche 1-10 oder die pharmazeutische Zusammensetzung nach Anspruch 11, zur Anwendung beim Induzieren einer Glukosetoleranz und/oder Verbessern der glykämischen Kontrolle oder zur Anwendung beim Reduzieren der Essensaufnahme und/oder Reduzieren des Körpergewichts bei einem Subjekt, das einem solchen Bedarf hat.

14. Verbindung nach Anspruch 1, zur Anwendung bei der Behandlung von Diabetes mellitus, Diabetes mellitus im Zusammenhang mit Adipositas, Adipositas, einer Essstörung oder einer Stoffwechselstörung bei einem Subjekt.

15. Verbindung nach einem der Ansprüche 1-10 oder die pharmazeutische Zusammensetzung nach Anspruch 11, zur Verwendung als Medikament.

16. Verbindung nach Anspruch 1, wobei R₂ -SO₃H an Position 2 des Fluorenrings ist und wobei der PEG-Rest ein PEG von 40 kDa ist.

## Revendications

1. Composé de formule (X)n-Y, dans laquelle Y est un double agoniste du récepteur GLP-1/Glucagon constitué d'un peptide d'oxyntomoduline (OXM) ayant la séquence d'acides aminés de SEQ ID NO: 1 et portant un groupe amino ou hydroxyle libre, et dans laquelle X est un radical de formule (i) 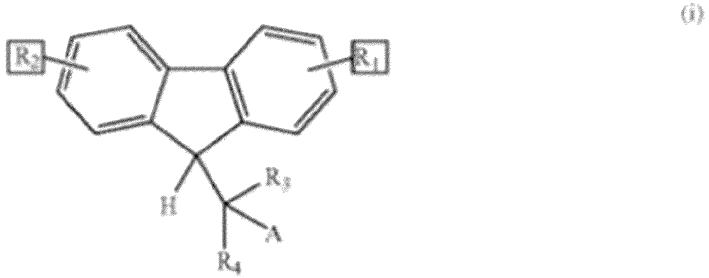 dans laquelle
R₁ est un fragment de polyéthylène glycol (PEG) ;
R₂ est soit un hydrogène, soit un groupe -SO₃H en position 2 du cycle fluorène ;
R₃ et R₄ représentent chacun de l'hydrogène ;
A est -OCO-, dans lequel le radical de la formule (i) est lié à un groupe amino ou hydroxyle du médicament Y ; et
n est un entier d'au moins un.

2. Composé selon la revendication 1, **caractérisé en ce que** le fragment PEG est un PEG linéaire.

3. Composé selon la revendication 1, **caractérisé en ce que** le fragment PEG est un PEG ramifié.

4. Composé selon la revendication 1, **caractérisé en ce que** le fragment PEG est un PEG de 30 kDa, de 40 kDa ou de 60 kDa.

5. Composé selon la revendication 1, **caractérisé en ce que** ledit composé est préparé à partir d'un MAL-Fmoc-NHS répondant à la formule : 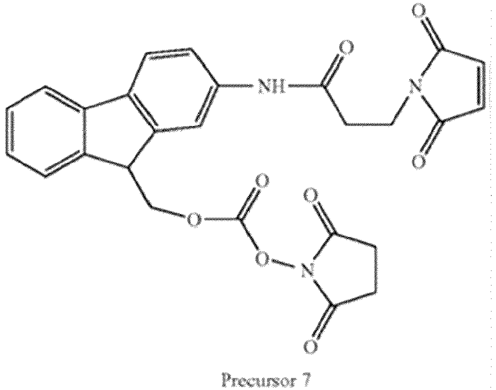 où ledit composé est désigné par (PEG-Fmoc-)n-OXM.

6. Composé selon la revendication 1, **caractérisé en ce que** ledit composé est préparé à partir d'un MAL-FMS-NHS répondant à la formule : 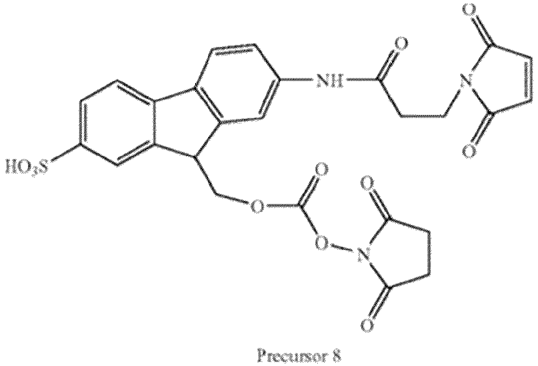 où ledit composé est désigné par (PEG-FMS-)n-OXM.

7. Composé selon la revendication 5, **caractérisé en ce que** Y est lié au radical Fmoc par l'intermédiaire d'un groupe amino.

8. Composé selon la revendication 6, **caractérisé en ce que** Y est lié au radical FMS par l'intermédiaire d'un groupe amino.

9. Composé selon les revendications 7 ou 8, **caractérisé en ce que** ledit groupe amino est l'extrémité amino de ladite oxyntomoduline.

10. Composé selon les revendications 7 ou 8, **caractérisé en ce que** le PEG est lié au radical FMS ou Fmoc par l'intermédiaire d'un groupe sulfhydryle.

11. Composition pharmaceutique comprenant un composé selon l'une quelconque des revendications 1 à 10, et un excipient pharmaceutiquement acceptable.

12. Procédé de préparation d'oxyntomoduline ayant une demi-vie biologique prolongée, consistant en l'étape de conjugaison de l'oxyntomoduline, d'un polymère de polyéthylène glycol (polymère PEG) et de 9-fluorénylméthoxycarbonyle (Fmoc) ou de 2-sulfo-9 fluorénylméthoxycarbonyle (FMS) dans un rapport molaire de 1:1:0,5 à 1:1:3,5, pour former un composé selon la revendication 1.

13. Composé selon l'une quelconque des revendications 1 à 10, ou composition pharmaceutique selon la revendication 11, destiné à être utilisé pour induire une tolérance au glucose et/ou améliorer le contrôle glycémique, ou pour réduire la prise alimentaire et/ou réduire le poids corporel, chez un sujet qui en a besoin.

14. Composé selon la revendication 1, destiné à être utilisé dans le traitement du diabète sucré, du diabète sucré associé à l'obésité, de l'obésité, d'un trouble de l'alimentation ou d'un trouble métabolique, chez un sujet.

15. Composé selon l'une quelconque des revendications 1 à 10, ou composition pharmaceutique selon la revendication 11, destiné à être utilisé comme médicament.

16. Composé selon la revendication 1, **caractérisé en ce que** R₂ est - SO₃H en position 2 du cycle fluorène, et le fragment PEG est un PEG de 40 kDa.
